# EUROPÄISCHE PATENTANMELDUNG

(11) **EP 1 230 917 A1**
(43) Veröffentlichungstag der Anmeldung: **14.08.2002**
(21) Anmeldenummer: 02002054.1
(22) Anmeldetag: 08.02.2002
(51) Int. Cl.: A61K 9/127, A61K 38/13

(54) **Invasomen zur Therapie von Erkrankungen, ihre Herstellung und Verwendung**

(30) Priorität: 08.02.2001 DE 10105659
(71) Anmelder: Vectron Therapeutics AG, 35037 Marburg (DE)
(72) Erfinder: Fahr, Alfred, 35091 Cölbe (DE); Müller, Rolf, 35037 Marburg (DE)
(74) Vertreter: Bösl, Raphael, Dr. rer. nat., Dipl.-Chem.

(57) **Zusammenfassung**

Die vorliegende Erfindung betrifft Invasomen, enthaltend ein Lipidgemisch umfassend ein oder mehrere Lipide, vorzugsweise neutrale Lipide, und ein oder mehrere Lysophosphatide und mindestens einen pharmakologischen Wirkstoff, vorzugsweise ein Immunmodulanz, deren Herstellung und deren Verwendung zur Therapie von Erkrankungen, vorzugsweise von Erkrankungen, die durch eine Modulation des Immunsystems behandelbar sind.

## Beschreibung

Die vorliegende Erfindung betrifft Invasomen, enthaltend ein Lipidgemisch umfassend ein oder mehrere Lipide, vorzugsweise neutrale Lipide, und ein oder mehrere Lysophosphatide und mindestens einen pharmakologischen Wirkstoff, vorzugsweise ein Immunmodulanz, deren Herstellung und deren Verwendung zur Therapie von Erkrankungen, vorzugsweise von Erkrankungen die durch eine Modulation des Immunsystems behandelbar sind.

Die topische Applikation hydrophiler oder hydrophober pharmakologisch wirksamer Substanzen mit dem Ziel den jeweiligen Wirkstoff in tiefere Hautschichten oder sogar durch die Haut hindurch zu transportieren ist ein schwieriges Unterfangen, da die oberste Schicht der Haut (Stratum corneum) für die meisten Substanzen undurchlässig ist. Für viele Wirkstoffe wäre es jedoch wünschenswert, diese direkt durch die Haut zu applizieren. Beispielsweise ist die transdermale Verabreichung von Insulin für Diabetiker interessant, da dadurch die täglichen Insulinspritzen entfallen würden. Während ein solcher effizienter transdermaler Transport für alle Substanzen wünschenswert ist, die systemische Wirkung entfalten sollen, ist er nicht für Substanzen geeignet, deren Wirkung auf die tieferen Schichten der Haut beschränkt bleiben sollte. Dies gilt beispielsweise für Wirkstoffe, die bei systemischer Anwendung starke Nebenwirkungen und/oder Toxizität zeigen, die aber dennoch zur Therapie bestimmter Hauterkrankungen gut geeignet sind.

In der Literatur ist eine Vielzahl von Versuchen beschrieben worden, die Barriere der Haut zu überwinden. Besondere Aufmerksamkeit haben hierbei Liposomen erlangt. Mezei und Gulasekharam (Life Sci. 26: 1473-1777, 1980) waren die ersten, die berichteten, dass Liposomen, die mit Triamcinolonacetonid beladen waren eine 3- bis 5-fache Anhäufung des Wirkstoffs in der Epidermis und Dermis der Haut ermöglichten. Vanlerberghe et al. (Coll Nat. CRNS: 938-942, 1978) zeigten, dass die Verwendung von Vesikeln aus nicht-ionischen oberflächenaktiven Substanzen (Niosomen) die Penetration von Natriumpyrrolidoncarboxylat durch das Stratum corneum erhöhten. WO 92/03122-A1 offenbarte die Verwendung von sogenannten "Transferosomen", um Insulin systemisch zu verabreichen. Die Transferosomen bestehen aus einem Lipidanteil und einer randaktiven Substanz. Die Transferosomen werden auch als "flexible" Liposomen bezeichnet, die im Gegensatz zu Liposomen mit rigideren Strukturen in der Lage sind, Substanzen den Weg durch das Stratum corneum zu erleichtern. Diese Versuche zielten jedoch in erster Linie darauf, die jeweiligen Wirkstoffe möglichst effizient durch die Haut in den Körper zu transportieren.

Auch bei Hauterkrankungen, an denen Zellen in tiefer liegenden Hautschichten, wie beispielsweise Zellen in der Stratum germinativum, beteiligt sind, ist es wünschenswert Wirkstoffe effizient durch die Barriere des Stratum corneums zu transportieren. Ein wichtiges Beispiel hierfür sind Hauterkrankungen, an denen das Immunsystem der Haut beteiligt ist. Das Immunsystem der Haut bilden zum einen die in der Haut bereits vorhandenen Zellen, wie beispielsweise dendritische Zellen aber auch die jeweils bei Erkrankung, Infektion und/oder entzündlichen Prozessen in die Haut infiltrierenden Immunzellen, wie beispielweise T-Zellen, Langerhans-Zellen und Makrophagen. Bei diesen Hauterkrankungen ist beispielsweise eine gegenüber gesunder Haut erhöhte Infiltration bzw. Aktivierung der genannten Immunzellen zu beobachten, ohne dass tatsächlich eine Verwundung oder Infektion der Haut vorliegt. Es wird vermutet, dass viele dieser Erkrankungen, wie beispielsweise Alopecia areata, Psoriasis oder Neurodermitis, auch durch Autoimmunreaktionen hervorgerufen werden.

Alopecia areata ist eine relativ häufige Erkrankung für deren Ausbruch über die gesamte Lebenszeit eine Wahrscheinlichkeit von 1,7% in der Bevölkerung besteht (Safavi et al. Mayo Clin. Proc. 70: 628-633, 1995). Alopecia areata geht mit einem reversiblen, auf begrenzte Areale beschränkten Haarverlust einher, kann jedoch manchmal auch zum kompletten Haarverlust führen (Alopecia totalis). Histopathologische Kennzeichen von Alopecia areata beinhalten perifolliculare lymphozytische Infiltration, die die anagenen Haarfollikel betreffen, mit anschließender Miniaturisierung dieser Follikel (Golnik und Orphanos, in Orphanos und Happle (Editoren) Hair and hair diseases. Berlin, Springer Verlag, Seiten 529-569, 1990). Diese Infiltrate bestehen in erster Linie aus T4-Lymphozyten, Makrophagen und Langerhans-Zellen (Tobin et al. J. Invest. Dermatol. 109: 329-333, 1997). Der Haarverlust ist mit peri- und intrafollikularer inflammatorischer Infiltration der anagenen Haarfollikel assoziiert, wobei das Infiltrat in erster Linie aus CD4⁺ und CD8⁺ Zellen bestehen. Die jeweilige Funktion dieser Zellen in der Pathogenese der Alopecia areata ist bisher unverstanden (Mc Elwee et al. B.J. Dermatol. 140: 432-437, 1999). Die beobachtete peribulbare und intrabulbare Anhäufung von T-Lymphozyten (Perret et al., Acta Drem. Venerol. 64:26-30, 1984) und eine fehlgeregelte Expression des "intercellular adhesion molecule 1" (ICAM -1) und des HLA-DR Moleküls auf follikulären Keratinozyten und dermalen Papillen (Hamm et al., Arch. Dermatol. Res. 280:179-181, 1988; Nickoloff & Griffiths, J. Invest. Dermatol. 96:91-92, 1991) deuten jedoch darauf hin, dass das Immunsystem der Haut an der Erkrankung beteiligt ist (Baadsgaard Dermatol. 96:89-89, 1991).

Zur Behandlung dieser Hauterkrankungen bieten sich deshalb immunmodulierende Substanzen an, die beispielsweise die Immunantwort unterdrücken. Solche Substanzen, die bisher vor allem zur systemischen Behandlung dieser Hauterkrankungen verwendet wurden, sind beispielsweise Cyclosporin A oder FK 506. Der Nachteil der systemischen Anwendung immunmodulatorischer Substanzen liegt beispielsweise bei Cyclosporin A in der Nephrotoxizität, eine Nebenwirkung, die bei der Verwendung von Cyclosporin zur Vermeidung von Transplantatabstoßungsreaktionen tolerierbar ist, jedoch nicht bei der Behandlung nicht unmittelbar lebensbedrohender Hauterkrankungen.

Die im Stand der Technik beschriebenen Versuche, die systemische Gabe von Immunsuppressiva durch topische Applikation zu vermeiden, waren jedoch bisher nicht erfolgreich. Die verwendeten Verfahren führten entweder zu einem ungenügenden Transport durch das Stratum corneum oder führten zu einer unerwünschten Erhöhung der Wirkstoffkonzentration im Serum, d.h. systemischer Wirkung. Beispielsweise zeigte Griffiths et al. (Lancet I: 806, 1987), dass eine Lösung, die aus 2% Cyclosporin A in Unguentum Merk hergestellt wurde, zu keinerlei Effekt bei der Behandlung von Psoriasis führte. Black et al. (J. Inves. Dermatol. 5: 644-648, 1990) verwendeten neben systemisch verabreichtem Cyclosporin ein öliges mit Cyclosporin beladenes Vehikel um die T-Zell-vermittelte Immunantwort in der Haut zu verringern. Sie beobachteten eine verringerte T-Zell-vermittelte Immunantwort, beobachteten jedoch auch nach Beendigung der systemischen Gabe und ausschließlicher topischer Gabe eine erhöhte systemische Cyclosporinmenge. Gleichermaßen hat Gilha et al. (Acta Derm. Medarol, 69: 252-253, 1989) mit einer 10% Cyclosporin A-Lösung in einer öligen Zubereitung keinen Effekt auf Alopecia areata gesehen, stellt jedoch auch keine erhöhten Cyclosporin Level im Blut der Patienten fest. Niemiec et al. (Pharm. Res. 12:1184-1188, 1995) verwendeten drei verschiedene nicht-ionische Liposomen und untersuchten den Transport von Cyclosporin A in einem Hamsterohrmodell. Eine Einlagerung von Cyclosporin A wurde nur bei Verwendung von Liposomen beobachtet, die aus Glyceryldilaurat/Colesterol/Polyoxyethylen-10-Stearylether im Mischungs-verhältnis 57:15:28 Gew.-% bestanden. Eine auf Phospholipid basierende Liposomenzubereitung führte nur zu einer minimalen Aufnahme von Cyclosporin A in die Haut.

Guo et al. (Int. J. Farm. 194: 201-207, 2000) verwendeten die von Cevc et al. (Adv. Drug. Del. Rev. 18: 349-378, 1996 und in WO 92/03122) beschriebenen Transferosomen, um Cyclosporin durch die Haut zu transportieren. In dieser Studie wurde jedoch bereits 2 Stunden nach Anwendung der Transferosomen eine Serumkonzentration von 63 ng/ml Cyclosporin beobachtet. Die von Guo et al. verwendeten Vesikel wurden aus hochreinem Lecithin und dem Tensid Natriumcholat hergestellt. Die Autoren ziehen aus den Ergebnissen den Schluss, dass es möglich ist, Cyclosporin systemisch über die Haut zu verabreichen.

Der Mangel an geeigneten Transportsystemen, die es erlauben würden, den Effekt eines pharmakologischen Wirkstoffs, insbesondere eines immunmodulierenden Wirkstoffes auf die jeweils zu behandelnden Bereiche der Haut zu beschränken, zeigt sich auch darin, dass bei drei wichtigen Hauterkrankungen, die durch eine Modulation des Immunsystems behandelbar sind, d.h. bei aktiver Psoriasis, atopischer Dermatitis und Alopecia areata, die Behandlung immer noch mit systemisch verabreichten Cyclosporin A erfolgt (Iconomidou et al. (Dermatol. 199: 144-148, 1999; Naeyaert et al. Dermatol. 198: 145-152, 1999 und Ferando und Grimalt, Dermatol. 199: 86-69, 1999).

Da bei einer Reihe von Tieren Erkrankungen beschrieben worden sind, die dem Haarverlust der menschlichen Alopecia areata ähneln, ist diese Erkrankung geeignet, als Modellsystem zur Identifizierung geeigneter Transportsysteme zu dienen. Eine Form des reversiblen Haarverlustes, der klinisch und histopathologisch der humanen Alopecia areata entspricht, ist beispielsweise für DEBR-Ratten beschrieben worden (Sundberg et al., J. Invest. Dermatol. 104: 32-33, 1995). DEBR-Ratten entwickeln etwa 6 Monaten nach der Geburt die Symptome der Krankheit, wobei sich in einigen Ratten-Kolonien die Erkrankung nach 18 Monaten bei 20% aller Tiere durchgesetzt hat.

Im Rahmen der vorliegenden Erfindung wurde eine mit Cyclosporin A beladene Liposomenform, Invasomen genannt, gefunden, mit der der partielle Haarausfall der DEBR-Ratten erfolgreich behandelt werden konnte, ohne dass die Anwendung der Invasomen gleichzeitig zu einer messbaren Erhöhung der Cyclosporin A Menge im Serum führte.

Gegenstand der vorliegenden Erfindung ist deshalb ein Invasom, das ein Lipidgemisch umfassend ein oder mehrere Lipide und ein oder mehrere Lysophosphatide, wobei die Lysophosphatide an dem Lipidgemisch einen Anteil im Bereich von ca. 0,1 bis ca. 40 Gew.-% haben, und das mindestens einen pharmakologischen Wirkstoff, vorzugsweise ein Immunsuppressivum enthält.

Der Begriff "Invasom" bezeichnet ein unilamellares, bilamellares, oligolamellares (3, 4, 5, 6, 7, 8, 9, oder 10 Lamellen) oder multilamellares (mehr als 10 Lamellen) lipidhaltiges Vesikel, das nur eine geringe Menge des jeweilig enthaltenen pharmakologischen Wirkstoffs, insbesondere eines immunmodulierenden Wirkstoffs transdermal transportiert, so dass der pharmakologische Wirkstoff, insbesondere ein immunmodulierender Wirkstoff, nur eine geringe Konzentration im Serum des Patienten erreicht und deshalb keine oder nur eine geringe systemische Wirkung entfalten kann. Bei Verwendung der erfindungsgemäßen Invasomen in therapeutisch wirksamen Mengen kann nach der Applikation, insbesondere nach ca. 2 Stunden, ca. 4 Stunden, ca. 6 Stunden, ca. 9 Stunden, ca. 14 h Stunden, ca. 24 Stunden, ca. 35 Stunden oder nach ca. 48 Stunden höchstens ca. 50 ng/ml, insbesondere höchstens ca. 10 ng/ml besonders bevorzugt höchstens ca. 7 ng/ml und am meisten bevorzugt höchstens ca. 4 ng/ml des jeweiligen pharmakologischen Wirkstoffs, insbesondere des immunmodulierenden Wirkstoffs, im Serum des Patienten nachgewiesen werden. Vorzugsweise liegt die Serumkonzentration des pharmakologischen Wirkstoffs, insbesondere eines immunmodulierenden Wirkstoffs, ca. 2 Stunden nach Applikation unter der Nachweisgrenze des jeweiligen Wirkstoffs im Serum. Geeignete Nachweisverfahren sind abhängig vom verwendeten pharmakologischen Wirkstoff, insbesondere immunmodulierenden Wirkstoffs, beispielsweise HPLC, ELISA, RT-PCR, Scintilation, bei Verwendung radioaktiv markierter pharmakologischer Wirkstoffe, oder Gaschromatographie. Eine therapeutisch wirksame Menge der erfindungsgemäßen Invasomen ist beispielsweise für Invasomen, die auf das Gesamtgewicht der Invasomenmischung bezogen 0,5% Cyclosporin A enthalten, 20 bis 80 µl Invasomen/cm² behandelte Haut.

Die lamellare Struktur des erfindungsgemäßen Invasoms kann auch durch eine ungerade Anzahl von Lamellen, wie beispielsweise 1, 3, 5, 7, 9 oder 11 Lamellen gebildet werden, wobei eine Seite das hydrophobe Innere des Vesikels und die andere Seite das hydrophile Äußere bildet. Diese Art von Vesikel ist besonders geeignet, um beispielsweise hydrophobe pharmakologische Wirkstoffe, Immunsuppressiva aufzunehmen.

Die erfindungsgemäßen Invasomen enthalten das Lipidgemisch und den (die) pharmakologischen Wirkstoff(e), insbesondere das (die) immunmodulierenden Wirkstoff(e) in einem Gewichtsverhältnis im Bereich von ca. 1:1 bis ca. 1000:1 [Lipidgemisch: pharmakologischen Wirkstoff(e)], vorzugsweise im Bereich von ca. 2:1 bis ca. 100:1, bevorzugterweise im Bereich von ca. 5:1 bis ca. 50:1, noch bevorzugter im Bereich von ca. 15:1 bis ca. 30:1 und am meisten bevorzugt im Bereich von ca. 18:1 bis ca. 22:1.

Das im Invasom enthaltende Lipidgemisch stammt aus pflanzlichen Quellen, tierischen Quellen und/oder ist vollsynthetischen Ursprungs. Die Lipide sind vorzugsweise neutrale und/oder anionische Lipide. Der Anteil an neutralen bzw. anionischen Lipiden in einem aus pflanzlichen Quellen und/oder tierischen Quellen stammenden Lipidgemisch kann durch weitere Reinigungsverfahren, die auf die Anreicherung neutraler und/oder anionischer Lipide gerichtet sind, erhöht werden. Vorzugsweise haben die neutralen oder anionischen Lipide bzw. eine Mischung beider Lipidformen einen Anteil im Bereich von ca. 40 bis ca. 99,9 Gew.-% an der Gesamtmenge des Lipidgemischs Bevorzugt sind daher Lipidgemische aus pflanzlichen bzw. tierischen Quellen, die natürlicherweise neutrale und/oder anionische Lipide im angegebenen Bereich enthalten. Verfahren zur Gewinnung von Fetten und Ölen und zur Anreicherung einzelner Komponenten sind im Stand der Technik bekannt und beispielsweise in Uhlmanns Enzyklopädie der technischen Chemie, Band 11, Seiten 455-523, 1976 beschrieben. Der Begriff Lipidgemisch umfasst auch Gemische, die aus Lipiden verschiedener Quellen beispielsweise verschiedenen Pflanzen stammen, insbesondere, wenn das Gemisch neutrale und oder anionische Lipide im Bereich von ca. 40 bis ca. 99,9-Gew.-% enthält. In einer besonders bevorzugten Ausführungsform enthält das Lipidgemisch neutrale Lipide im Bereich von ca. 40 bis ca. 99,9-Gew.-%.

Ein Lipid im Sinne der vorliegenden Erfindung ist jede Substanz, die fettartige oder fettähnliche Eigenschaften besitzt und wobei diese Substanz über einen ausgedehnten apolaren Anteil und einen polaren, wasserlöslichen Anteil verfügen.

Dazu gehören beispielsweise neutrale, anionische oder kationische Lipide jedoch keine Lysophosphatide.

Ein Lipidgemisch im Sinne der vorliegenden Erfindung ist eine beliebige Mischung der vorgenannten Lipide die zusätzlich ein oder mehrere Lysophosphatide enthält

Ein neutrales Lipid im Sinne der vorliegenden Erfindung ist ein Lipid das ein oder mehrerer positive und negative Ladungsträger enthält, wobei in dem polaren Anteil jeweils die selbe Anzahl von positiven und negativen Ladungsträgern vorhanden ist. Daher weisen Invasomen, die neutrale Lipide enthalten, abhängig vom neutralen Lipid(en) im Bereich von ca. pH 6,0 bis ca. 8,0, vorzugsweise im Bereich von ca. pH 6,5 bis ca. 7,5 und besonders bevorzugt bei ca. pH 7,0 eine neutrale Gesamtladung auf.

Ein anionisches Lipid im Sinne der vorliegenden Erfindung ist ein Lipid das keine oder mehrerer positive und ein oder mehrere negative Ladungsträger enthält, wobei in dem polaren Anteil die Anzahl der negativen Ladungsträgern höher ist als die Anzahl der positiven Ladungsträger. Daher weisen Invasomen, die anionische Lipide enthalten, abhängig vom anionischen Lipid(en) im Bereich von ca. pH 6,0 bis ca. 8,0, vorzugsweise im Bereich von ca. pH 6,5 bis ca. 7,5 und besonders bevorzugt bei ca. pH 7,0 eine negative Gesamtladung auf.

Wenn das Lipidgemisch einer Mischung aus neutralen und anionischen Lipiden enthält, weisen die Invasomen eine negative Gesamtladung auf.

Ein weiterer Bestandteil der erfindungsgemäßen Invasomen sind ein oder mehrere Lysophosphatide, wobei der Anteil der Lysophosphatide an dem Lipidgemisch im Bereich von ca. 0,1 bis ca. 40 Gew.-%, vorzugsweise von ca. 1 bis ca. 25 Gew.-%, noch bevorzugter von ca. 4 bis ca. 15 Gew.-% und am bevorzugtesten von ca. 10 bis ca. 15 Gew.-% liegt.

Bevorzugte Lysophosphatide, die im Rahmen der vorliegenden Erfindung verwendet werden können sind beispielsweise Lysophosphatidylcholin (Lysolecithin), Lysophosphatidylethanolamin, Lysophosphatidylinositol, Lysophosphatidylserin, Monolysocardiolipin oder Dilysocardiolipin. Ein besonders bevorzugtes Lysophosphatide ist Lysolecithin. Natürlich gewonnene oder aufgereinigte Lipidgemische enthalten z.T. Lysophosphatide innerhalb den angegebenen Gewichtsbereiche. Andernfalls können natürlichen oder synthetischen Lipiden, vorzugsweise neutralen und/oder anionischen Lipiden, natürliche oder synthetische Lysophosphatide in den angegebenen Gewichtsbereichen zugesetzt werden.

Ein erfindungsgemäßes Invasom hat eine Größe zwischen 20 und 1000 nm. Die Größenverteilung der Invasomen ist von der Herstellungsmethode abhängig. So führt Sonifizierung der Lipidgemische zu kleinen Vesikeln, während "Vortexen" zu größeren Vesikel führt. Vesikel innerhalb einer bestimmten Größenklasse lassen sich durch im Stand der Technik bekannte Methoden wie Mikrofiltration oder Zentrifugation auswählen.

Die erfindungsgemäßen Invasom haben in einer bevorzugten Ausführungsform einen Durchmesser im Bereich von ca. 50 bis ca. 400 nm, insbesondere im Bereich von ca. 60 bis ca. 200 nm und besonders bevorzugt im Bereich von ca. 80 bis ca. 150 nm. Die Größe der Vesikel hat Einfluss auf die Permeationsfähigkeit der Vesikel, wobei kleinere Vesikel im Bereich von ca. 50 bis ca. 200 nm eine bessere Durchdringungsfähigkeit des Stratum corneums aufweisen, als größere Vesikel mit einem Durchmesser von beispielsweise ca. 600 bis ca. 800 nm.

Bevorzugte Quellen zur Gewinnung des Lipidgemischs, das zur Herstellung eines erfindungsgemäßen Invasoms verwendet werden kann, sind Lipidgemische, die aus Ölsaaten, insbesondere aus Sojabohnen, Kattunsamen, Kokosnusskern, Erdnuss, Saflorsamen, Sesamsamen, Sonnenblumensamen, Leinsamen, Raps, Weizenkeimen, Oliven oder tierischen Fetten, insbesondere Walfett, Stratum corneum Lipid, Rinderklauenöl und/oder Ei gewonnen wird. Eine besonders bevorzugte Quelle für Lipidgemische sind Sojabohnen. Eine Anreicherung der neutralen Lipide, anionischen Lipide und/oder Lysophosphatide in den aus diesen Ausgangsmaterialien gewinnbaren Lipidgemischen ist beispielsweise durch chromatographische Verfahren, wie HPLC und andere im Stand der Technik bekannte Verfahren möglich (siehe Uhlmann, 1976, Supra).

In einer bevorzugten Ausführungsform des erfindungsgemäßen Invasoms ist der Gewichtsanteil des neutralen, anionischen Lipids oder Gemisches beider Lipidformen, insbesondere des neutralen Lipids, an dem Lipidgemisch im Bereich von ca. 50 bis ca. 98 Gew.-%, vorzugsweise im Bereich von ca. 60 bis ca. 95 Gew.%, noch bevorzugter im Bereich von ca. 75 bis ca. 95 Gew.-% und am meisten bevorzugt ca. 90 Gew.-%. Eine Erhöhung des Anteils der neutralen, anionischen oder eines Gemisches beider Lipidformen über ca. 40% am Lipidgemisch führt zu einer Verbesserung der Permeationseigenschaften, die sich jedoch bei weiterer Erhöhung des Anteils an neutralen oder anionischen Lipid über 95 Gew.-% an dem Lipidgemisch wieder verschlechtern. So erlangt ein reines neutrales Lipid erst wieder durch den Zusatz von oberflächenaktiven Substanzen (siehe Guo et al., 2000 Supra und WO 92/03122), wie beispielsweise Tensiden, die Fähigkeit das Stratum corneum zu durchdringen, was aber andererseits dann auch zur Durchdringung der weiteren Hautschichten und letztlich zu einer systemischen Freisetzung des jeweiligen Wirkstoffes führt.

In einer weiteren bevorzugten Ausführungsform des erfindungsgemäßen Invasoms ist das neutrale Lipid ein Glycerophospholipid, insbesondere ein Phosphatidylcholin, ein Steroid, ein Glycerophosphonolipid, ein Glycerophosphinolipid und/oder ein Sphingolipid. Besonders bevorzugt ist hierbei jedoch ein Glycerophospholipid, insbesondere ein Phosphatidylcholin. Geeignete Phosphatidylcholin-haltige Lipidgemische sind beispielsweise mit einem Anteil von ca. 80 Gew.-% an der Gesamtlipidmischung kommerziell erhältlich. Phospholipon 80 (Nat 8539; Nattermann Phospholipid GmbH) ist ein solches Lipidgemisch, das auch als ethanolische Lösung erhältlich ist und die folgende Zusammensetzung hat: 73,0-79,0 Gew.-% (3-sn-Phosphatidyl)cholin, 6 Gew.-% (3-sn-Lysophosphatidyl)cholin, 7 Gew.-% Kephalin und 7 Gew.-% Phosphatidsäure.

In einer weiteren bevorzugten Ausführungsform des erfindungsgemäßen Invasoms ist das anionische Lipid ein Phosphatidylserin, ein Phosphatidylglycerol, eine Phosphatidylsäure, Phosphatidylinositol und/oder ein Cholesterolglutarat.

Ein weiterer Gegenstand der Erfindung ist ein Invasom, das zusätzlich ein oder mehrere Terpene enthält. Terpene im Sinne der vorliegenden Erfindung umfassen Polymerisationsprodukte des Kohlenwasserstoffs Isopren. Nach der Anzahl der Isopren-Reste unterscheidet man Monoterpene (C₁₀), Sesquiterpene (C₁₅), Diterpene (C₂₀), Sesterpene (C₂₅), Triterpene (C₃₀), Tetraterpene (C₄₀) und Polyterpene. Die aus den Isopren-Grundeinheiten gebildeten azyklischen Kohlenwasserstoffe können beispielsweise durch anschließende Substitutionen, Oxidation, Zyklisierungen und Umlagerung in eine Vielzahl von Verbindungen umgewandelt werden. Unter Terpenen im Sinne der vorliegenden Erfindung versteht man auch die abgeleiteten Alkohole, Ketone, Aldehyde und Ester.

Terpene werden dem Lipidgemisch im Bereich von ca. 0,1 bis ca. 200 Gew.-% bezogen auf die Menge des Lipidgemischs zugesetzt. Vorzugsweise jedoch im Bereich von ca. 5 bis ca. 100 Gew.-%, noch bevorzugter im Bereich von ca. 10 bis ca. 50 Gew.-% und am meisten bevorzugt im Bereich von ca. 15 bis ca. 30 Gew.-%. Wenn die zugesetzte Terpenmenge zu hoch wird, unterbinden die Terpene die Bildung der Invasomen. Die jeweilige Terpenmenge, die die Invasomenbildung behindert, hängt von dem verwendeten Lipidgemisch und dem verwendeten pharmakologischen Wirkstoff ab. Ob die gewählte Terpenmenge die Invasomenbildung behindert lässt sich leicht durch eines der nachfolgend beschriebenen Verfahren zur Herstellung von erfindungsgemäßen Invasomen testen. Messung der Anisotropie von Invasomen haben gezeigt, dass Invasomen, die Terpene enthalten, einer gegenüber nicht Terpen-haltigen Invasomen eine verringerte Flexibilität aufweisen.

In einer bevorzugten Ausführungsform enthält das erfindungsgemäße Invasom ein oder mehrere der folgenden Terpene: Cineol, Citral, Limonen, insbesondere D-Limonen, Menthan, Terpinen, Terpinolen, Menthol, insbesondere 1-Menthol, Carveol, insbesondere 1-Carveol, Menthon, Carvon, Pinen, insbesondere β-Pinen, Caren, insbesondere 3-Caren, Terpineol, Terpinen-4-ol, Pulegon, Piperiton, Cyclohexanoxid, Limonenoxyd, Pinenoxyd, Cyclopentenoxid, Ascaridol, 7-Oxybicyclo-[2.2.1]-heptan, Cymol, Camphen, Citronellol, Geraniol, Nerol, Linalool, Borneol, Thujol, Sabinol, Myrtenol, Thymol, Verbenol, Fenchol, Piperitol, Perillaaldehyd, Phellandral, Citronellal, Myrtenal, Piperiton, Thujon, Umbellolun, Verbenon, Chrysanthenon, Fenchon, Campher, Chinon, Menthofuran, Linalooloxid, Rosenoxid und/oder Quinghaosu, wobei die verschiedenen Struktur- und Konfigurationsisomere der vorangenannten Terpene umfasst sind.

Das Invasom kann einzelne Terpene oder Terpengemische enthalten. Besonders bevorzugt ist ein Invasom, das eine Mischung aus D-Limonen, Cineol und Citral enthält. Bevorzugte Mischungsverhältnisse für diese Terpene liegen im Bereich von ca. 2:49:49 bis ca. 1:1:1, vorzugsweise im Bereich von ca. 4:48:48 bis ca. 1:3:3 und besonders bevorzugt im Bereich von ca. 10:45:45 (D-Limonen:Cineol:Citral).

Pharmakologische Wirkstoffe im Sinne der vorliegenden Erfindung sind alle Substanzen, die eine therapeutische Wirkung hervorrufen, insbesondere jedoch Substanzen, die in der Therapie von Hauterkrankungen eingesetzt werden. Daher sind bevorzugte pharmakologische Wirkstoffe Immunsuppressiva, Immunstimulantien, Antibiotika, Antiinfektiva, Antiallergika, Zytostatika, Zytotoxika, Mitogene, Chemokine, Cytokine, Dermatika und/oder physiologische oder pharmakologische Inhibitoren der Mitogene, Chemokine, Cytokine. Besonders bevorzugt pharmakologische Wirkstoffe sind Immunsuppressiva und Immunstimulantien.

Der Begriff "Immunsuppressivum" umfasst alle Substanzen, die die Funktion von Zellen beeinflussen, die an der Vermittlung der Immunantwort direkt oder indirekt beteiligt sind und wobei die Beeinflussung zu einer Verringerung der Immunantwort führt. Zu diesen Zellen gehören beispielsweise Makrophagen, dendritische Zellen, Langerhans-Zellen, indeterminierte Zellen, aber auch Zellen, die nicht zum Immunsystem selbst gehören, die aber an Immunerkrankungen der Haut beteiligt sind, wie Fibroblasten, Keratinozyten und Melanozyten. Die Stärke der Immunantwort lässt sich beispielsweise durch die Menge der produzierten Zytokine (wie Interferon-gamma), dem Nachweis von Aktivierungsmarkern auf dentritischen Zellen (wie MHCII oder CD86) oder die Anzahl der aktivierten CD8-positiven T-Zellen in der Haut bestimmen.

In einer bevorzugten Ausführungsform des erfindungsgemäßen Invasoms ist das Immunsuppressivum ein Glucokorticoid, insbesondere Beclomethason, Betamethason, Clocortolon, Cloprednol, Cortison, Dexamethason, Fludrocortison, Fludroxycortid, Flumetason, Fluocinolonacetonid, Fluocinonid, Fluocortolon, Fluormetholon, Fluprednidenacetat, Hydrocortison, Paramethason, Prednisolon, Prednison, Prednylidin, Pregnenolon, Triamcinolon oder Triamcinolonacetonid, ein Cylosporin, insbesondere Cyclosporin A, Mycophenolatmofetal, Tacrolimus, Rapamycin, FK 506, Cycloheximid-N-etylethanoat, Azathioprin, Ganciclovir, ein Anti-Lymphozytenglobulin, Ascomycin, Myriocin, ein pharmakologischer Inhibitor von MAP-Kinasen (insbesondere ein p38 Inhibitor wie VX-745) und/oder Methotrexat ist. Ein besonders bevorzugtes Invasom der vorliegenden Erfindung enthält Cyclosporin A als Immunsuppressivum.

Der Begriff "Immunstimulanz" umfasst alle Substanzen, die die Funktion von Zellen beeinflussen, die an der Vermittlung der Immunantwort direkt oder indirekt beteiligt sind und wobei die Beeinflussung zu einer Erhöhung der Immunantwort führt. Zu diesen Zellen gehören beispielsweise Makrophagen, Langerhans-Zellen und andere dendritische Zellen, Lymphozyten, indeterminierte Zellen, aber auch Zellen, die nicht zum Immunsystem selbst gehören, die aber an Immunerkrankungen der Haut beteiligt sind, wie Fibroblasten, Keratinozyten und Melanozyten, insbesondere jedoch Langerhans-Zellen. Die Stärke der Immunantwort lässt sich beispielsweise durch die Menge der produzierten Zytokine (wie Interferon-gamma), dem Nachweis von Aktivierungsmarkern auf dendritischen Zellen (wie MHCII oder CD86) oder die Anzahl der aktivierten CD8-positiven T-Zellen in der Haut bestimmen. Immunstimulanzien im Sinne der vorliegenden Erfindung sind insbesondere pflanzliche Immunstimulanzien, die beispielsweise aus *Echinacea palida* oder *Echinacea purpurea* gewonnen werden, Zytokine, wie beispielsweise Interleukine, Interferone und Kolonie-stimulierende Faktoren sowie bakterielle Bestandteile bzw. Moleküle, die diese nachahmen [wie bakterielle DNA und unmethylierte Oligodesoxynukleotide mit CpG-Sequenzen sowie Bestandteile der bakteriellen Zellwand oder Hülle, insbesondere Lipopolysaccharide und davon abgeleitete Moleküle wie Monophosphoryl-Lipid A, Muramyldipeptid (N-acetylmuramyl-L-alanyl-D-isoglutamine), und/oder PamCys3 sowie andere Moleküle wie Tetanus toxoid, Poly-L-Arginin oder MHCII-Peptide].

Der Begriff "Antibiotika" umfasst beispielweise Penicilline, Cephalosporine, Tetracycline, Aminoglykoside, Makrolid-Antibiotika, Lincosamide, Gyrasehemmer, Sulfonamide, Trimethoprim, Polypeptid-Antibiotika, Nitroimidazol-Derivate, Amphenicol, insbesondere Actinomycin, Alamethicin, Alexidin, 6-Aminopenicillan Säure, Amoxicilin, Amphotericin, Ampicilin, Anisomycin, Antiamoebin, Antimycin, Aphidicolin, Azidamfenicol, Azidocillin, Bacitracin, Beclomethason, Benzathin, Benzylpenicillin, Bleomycin, Bleomycinsulfat, Calcioum Ionophor A23187, Capreomycin, Carbenicillin, Cefacetril, Cefaclor, Cefamandol Nafat, Cefazolin, Cefalexin, Cefaloglycin, Cefaloridin, Cefalotin, Cefapirin, Cefazolin, Cefoperazon, Ceftriaxon, Cefuroxim, Cephalexin, Cephaloglycin, Cephalothin, Cephapirin, Cerulenin, Chloramphenicol, Chlortetracyclin, Chloramphenicol diacetat, Ciclacilin, Clindamycin, Chlormadinonacetat, Chlorpheniramin, Chromomycin A3, Cinnarizin, Ciprofloxacin, Clotrimazol, Cloxacillin, Colistinmethansulfonat, Cycloserin, Deacetylanisomycin, Demeclocyclin, 4,4'-Diaminodiphenyl sulfon, Diaveridin, Dicloxacillin, Dihydrostreptomycin, Dipyridamol, Doxorubicin, Doxycyclin, Epicillin, Erythromycin, Erythromycinstolat, Erythromycinthylsuccinat, Erythromycinstearat, Ethambutol, Flucloxacillin, Fluocinolonacetonid, 5-Fluorcytosin, Filipin, Formycin, Fumaramidomycin, Furaltadon; Fusidsäure, Geneticin, Gentamycin, Gentamycinsulfat, Gliotoxin, Gfamicidin, Griseofulvin, Helvolsäure, Hemolysin, Hetacillin, Kasugamycin, Kanamycin (A), Lasalocide, Lincomycin, Magnesidin, Melphalan, Metacyclin, Meticillin, Mevinolin, Micamycin, Mithramycin, Mithramycin A, Mithramycin Komplex, Mitomycin, Minocyclin, Mycophenolsäure, Myxothiazol, Natamycin, Nafcillin, Neomycin, Neomycinsulfat, 5-Nitro-2-furaldehydesemicarbazon, Novobiocin, Nystatin, Oleandomycin, Oleandomycinphosphat, Oxacihin, Oxytetracyclin, Paromomycin, Penicillin, Pecilocin, Pheneticillin, Phenoxymethylpenicillin, Phenylaminosalicylat, Phleomycin, Pivampicillin, Polymyxin B, Propicillin, Puromycin, Puromycinaminonucleosid, Puromycinaminonucleosid 5'-monophosphat, Pyridinolcarbamat, Rolitetracyclin, Rifampicin, Rifamycin B, Rifamycin SV, Spectinomycin, Spiramycin, Streptomycin, Streptomycinsulfat, Sulfabenzamide, Sulfadimethoxin, Sulfamethizol, Sulfamethoxazol, Tetracyclin, Thiamphenicol, Tobramycin, Troleandomycin, Tunicamycin, Tunicamycin A1-Homolog, Tunicamycin A2-Homolog, Valinomycin, Vancomycin, Vinomycin A1, Virginiamycin M1, Viomycin und/oder Xylostasin.

Der Begriff "Antiinfektiva" umfasst beispielsweise Antimykotica, Wirkstoffe mit antiparasitärer Wirkung und Virustatika, insbesondere, Amphotericin, Vifanozol, Buclosamid, Chinolinesulfat Chlormidazol, Chlorphenesine, Chlorquinaldol, Clodantoin, Cloxiquin, Cyclopiroloxamin, Dequaliniumchlorid, Dimazol, Fenticlor, Flucytosin, Griseofulvin, Ketoconazol, Miconazol, Natamycin, Sulbentin, Tioconazol, Tolnaftat, antiretrovirale Wirkstoffe und/oder Herpesmittel.

Der Begriff "Antiallergika" umfasst beispielweise Substanzen aus der Klasse der Globuline, Korticoide oder Antihistaminica, insbesondere Beclometason-, Betametasonecortison-, Dexametasone-Derivate, Bamipinacetat, Buclizin, Clemastin, Clemizol, Cromoglicinsäure, Cyproheptadin, Diflucorolonvalerat, Dimetotiazin, Diphenhydramin, Diphenylpyralin, Ephedrin, Fluocinolan, Histapyrrodin, Isothipendyl, Methadilazin, Oxomemazin, Paramethason, Predniliden, Theophillin und/oder Tolpropamintritoqualin.

Die Begriffe "Zytostatika" und "Zytotoxika" umfassen beispielweise alkylierende Substanzen, Antibiotika, Platinverbindungen, Hormone und Antihormone, Interferone sowie Inhibitoren zellzyklusabhängiger Proteinkinasen (CDKs) insbesondere Acediasulfon, Scriflaviniumchlorid, Ambazon, Dapson, Dibrompropamidin, Furazolidon, Hydroxymethyinitrofurantoin, Idoxuridin, Mafenid Sulfateolamid, Mepacrin, Metronidazol, Nalidixinsäure, Nifuratel, Nifuroxazid, Nifuarazin, Nifurtimox, Ninorazol, Nitrofurantoin, Oxolinsäure, Pentamidin, Phenazopyridin, Phthalylsulfatehiazol, Pyrimethamin, Salazosulfapyridin, Sulfacarbamid, Sulfacetamid, Sulfachlopyridazin, Sulfadiazin, Sulfadicramid, Sulfadimethoxin, Sulfaethidol, Sulfafurazol, Sulfaguanidin, Sulfaguanol, Sulfamethizol, Sulfamethoxazol, Cotrimoxazol, Sulfamethoxydiazin, Sulfamethoxypyridazin, Sulfamoxol, Sulfanilamid, Sulfaperin, Sulfaphenazol, Sulfatehiazol, Sulfisomidin, Tinidazol, Trimethoprim, Aclarubicin, Azathioprin, Bleomycin, Busulfan, Calciumfolinat, Carboplatin, Carmustin, Chloambucil, cis-Platin, Cyclophosphamid, Cyt-Arabin, Daunorubicin, Epirubicin, Fluoruracil, Fosfestrol, Hydroxycarbamid, Ifosfamid, Lomustin, Melphalan, Mercaptopurin, Methotrexate, Mitomycin C, Mitopodozid, Mitramicyn, Nimustin, Pipobroman, Prednimustin, Procarbazin, Testolacton, Theosulfane, Thiotepa, Tioguanin, Triaziquon, Trofosfamid, Vincristin, Vindesin, Vinblastin, Zorubicin, Flavopiridol, Oleomucin und/oder Preussin.

Die Begriffe "Mitogene" "Chemokine" und "Cytokine" umfassen beispielsweise Interferon-alpha, Interferon-beta, Interferon-gamma, Interleukin-1, Interleukin-2, Interleukin-7, Interleukin-10, Interleukin-12, Interleukin-18, GM-CSF, MIP-1-alpha/beta, RANTES, EGF, basischer oder saurer FGF, PDGF, IGF, VEGF, TGF-beta und/oder TNF-alpha..

Der Begriff "Dermatika" umfasst beispielweise Schieferölsulfonate, Teer und Teerderivate, Adstringientia, Antihidrotika, Aknemittel, Antipsoriatika, Antiseborrhoika und/oder Enzympräparate zur Behandlung von Hautdefekten.

In einer Ausführungsform ist der pharmakologische Wirkstoff eine Nukleinsäure, ein Protein, ein Peptid, ein Zucker und/oder ein Lipid beispielsweise bakterielle DNA, unmethylierte Oligodesoxynukleotide mit CpG-Sequenzen, Lipopolysaccharide Lipid A, Muramyldipeptid und/oder PAMCys3.

In einer bevorzugten Ausführungsform ist die Nukleinsäure ein Antisense-Oligonucleotid (Agrawal & Zhao, Curr. Opin. Chem. Biol. 2: 519-28, 1998), eine Antisense-RNA (Weintraub, Sci. Am. 262: 40-6, 1990), eine RNAi (Carthew, Curr. Opin. Cell. Biol. 13: 244-8, 2001), eine siRNA (Elbashir et al. Nature 411: 494-6, 2001) oder ein eine Triple Helix ausbildendes Oligonucleotid (Casey & Glazer, Prog. Nucleic Acid Res. Mol. Biol. 67: 163-92, 2001).

In einer weiteren Ausführungsform kodiert die Nukleinsäure für mindestens ein pharmakologisch wirksames Peptid und/oder Protein, insbesondere für ein immunmodulierendes Peptid oder Protein. Dabei bedeutet Peptid eine Aminosäurekette von 2 bis 50 Aminosäuren Länge und Protein eine Aminosäurekette die länger als 50 Aminosäuren ist. In diesem Fall werden die im Invasom enthaltenen Nukleinsäuren in Hautzellen eingeführt, ein solches Einführen wird auch als Transfektion bezeichnet. Geeignete Nukleinsäuren kodieren beispielsweise für Interferon-alpha, Interferon-beta, Interferon-gamma, Interleukin-1, Interleukin-2, Interleukin-7, Interleukin-10, Interleukin-12, Interleukin-18, GM-CSF, MIP-1-alpha/beta, RANTES, TGF-beta, TNF-alpha. und/oder CTLA4-Ig (siehe z.B. Abrams et al. J Clin Invest 103: 1243-52, 1999) . Die Erfindung umfasst auch Nukleinsäuren, die für die vorangenannten Peptide oder Proteine kodieren, deren Aminosäuresequenz jedoch Additionen, Substitutionen, Deletionen oder Mutationen einzelner Aminosäuren oder Aminosäuregruppen aufweist. Bevorzugt sind dabei sogenannte konservative Mutationen wie beispielsweise der Austausch eines Glutaminsäurerest gegen einen Asparaginsäurerest, Deletion oder Substitution von Bereichen des Proteins, die nicht für die pharmakologische, insbesondere immunmodulierende Wirkung verantwortlich sind. Die Nukleinsäuren können beispielsweise als Einzelstrang- oder Doppelstrang-DNA oder als RNA vorliegen, vorzugsweise jedoch als doppelsträngige DNA.

Die Nukleinsäure kann Teil eines Vektors sein, wobei Vektoren bevorzugt sind, die bei Transfektion einer Zelle zu einer dauerhaften Expression des pharmakologischen Wirkstoffs, insbesondere immunmodulierender Wirkstoff führen, wie beispielsweise episomal oder extrachromosomal replizierende Vektoren.

Die Nukleinsäure kann zusätzlich 5' oder 3' vom offenen Leserahmen, der für den pharmakologischen Wirkstoff, insbesondere das immunmodulierende Peptid oder Protein kodiert, Regulationselemente enthalten. Für die Expression sind beispielsweise Regulationselemente geeignet, die eine konstitutive, regulierbare, gewebsspezifische, zellzyklusspezifische oder metabolischspezifische Expression in eukaryotischen Zellen erlauben. Regulationselement gemäß der vorliegenden Erfindung umfassen Promotor-, Aktivator-, Enhancer-, Silencer und/oder Repressor-Sequenzen.

Beispiel für geeignete Regulationselemente, die konstitutive Expression in Eukaryonten ermöglichen, sind Promotoren, die von der RNA Polymerase III erkannt werden oder virale Promotoren, CMV-Enhancer, CMV-Promotor, SV40 Promotor oder LTR-Promotoren z. B. von MMTV (mouse mammary tumour virus; Lee et al. (1981) Nature 214, 228-232) und virale Promotor- und Aktivatorsequenzen, die beispielsweise aus HBV, HCV, HSV, HPV, EBV, HTLV oder HIV abgeleitet sind.

Beispiele für Regulationselemente, die induzierbare Expression in Eukaryonten ermöglichen, sind der Tetracyclinoperator in Kombination mit einem entsprechenden Repressor (Gossen et al. Curr. Opin. Biotechnol. 5, 516-20, 1994).

Beispiele für Regulationselemente, die gewebsspezifische Expression in Eukaryonten ermöglichen, sind Promotoren oder Aktivatorsequenzen aus Promotoren oder Enhancern von solchen Genen, die für Proteine kodieren, die nur in bestimmten Zelltypen, vorzugsweise Zellen des Immunsystems, exprimiert werden. Im Stand der Technik ist eine Vielzahl von Immunzell-spezifischen Regulationselementen beschrieben worden, die alle geeignet sind, die Expression des immunmodulierenden Wirkstoffs zu regulieren, besonders bevorzugt sind jedoch in T-Zellen aktive Promotoren oder in T-Zellen aktive Promotorfragmente, wie beispielsweise Promotoren oder Promotorfragmente der folgenden Gene: CD4, CD8 (beschrieben z.B. in Ellmeier et al. Annu. Rev. Immunol. 17: 523-54, 1999), IL-3, IL-4, IL-5, IL-13, GM-CSF (beschrieben z.B. in De Boer et al. Int. J. Biochem. Cell Biol. 31: 1221-36, 1999) oder synthetische Promotoren mit NF-AT Bindestellen (beschrieben z.B. in Hooijberg et al. Blood 96: 459-466, 2000). Besonders geeignete Promotoren sind nur in aktivierten T-Zellen aktiv und erlauben es dadurch, die Wirkung des(r) immunmodulierenden Wirkstoffs(e) auf die aktivierten T-Zellen selbst und ihre unmittelbare Umgebung zu beschränken.

In einer bevorzugten Ausführungsform ist das erfindungsgemäße Invasom in der Lage, therapeutisch wirksame Mengen mindestens eines pharmakologischen Wirkstoffs, insbesondere eines immunmodulierenden Wirkstoffs durch das Stratum corneum der Haut zu transportieren. Der Übergang vom Stratum corneum zu tieferen Hautschichten, wie beispielsweise dem Stratum granulosum, dem Stratum spinosum und dem Stratum basale, lässt sich histologisch bestimmen. Beispielsweise ist der Übergang von platten, kernlosen und verhornten Zellen zu abgeplatteten Körnerzellen für das Ende des Stratum corneums und den Beginn des Stratum granulosums charakteristisch. Die Methoden und Kriterien zur histologischen Bestimmung dieser Übergänge sind dem Fachmann bekannt. Die Verteilung des pharmakologischen Wirkstoffs, insbesondere Immunsuppressivums in der Haut lässt sich beispielsweise mit einem Klebestreifenabziehverfahren (Michel et al., Int. J. Pharm 84: 93-105, 1992) bestimmen. Hierbei wird die Haut nach Anwendung der erfindungsgemäßen Invasomen Schicht um Schicht mit Klebestreifen abgetragen und dann die Menge des pharmakologischen Wirkstoffs, insbesondere eines immunmodulierenden Wirkstoffs, in Abhängigkeit vom Abstand zur Hautoberfläche bestimmt. Die Bestimmung der therapeutischer Wirkung kann beispielsweise für Immunsuppressiva wie im Beispiel 2 erfolgen, in dem auf zwei angrenzende Hautareale eines Patienten oder eine Tieres, die von einer Hauterkrankung betroffen sind, die durch eine Modulation des Immunsystems der Haut behandelbar ist, jeweils Invasomen die einen immunmodulierenden Wirkstoff oder Invasomen die keinen immunmodulierenden Wirkstoff enthalten, aufgetragen werden. Die therapeutische Wirkung kann über einen Rückgang klinischer Parameter Änderung der Menge der in der Haut exprimierten Zytokine und/oder die Änderung der Anzahl der Immunzellen bzw. aktivierten Immunzellen in der Haut bestimmt werden. Bei Verwendung eines Immunsuppressivums kann die therapeutische Wirkung beispielsweise über eine Verringerung von Rötung, Schwellung und/oder Erwärmung oder durch die Verringerung der Menge der in der Haut exprimierten Zytokine und/oder die Verringerung der Anzahl der Immunzellen bzw. aktivierten Immunzellen in der Haut bestimmt werden. Bei der Verwendung eines Immunsuppressivums als pharmakologischer Wirkstoff liegt eine therapeutische Wirkung vor, wenn die Expression des jeweils untersuchten Zytokins in der Haut, die mit Invasomen enthaltend ein Immunsuppressivum behandelte wurde, gegenüber der Haut, die mit Invasomen ohne Immunsuppressivum behandelte wurde, um mindestens das ca. 2-fache, vorzugsweise um mindestens das ca. 5-fache, bevorzugter um mindestens das ca. 10-fache und am meisten bevorzugt um mindestens das ca. 20-fache verringert ist oder wenn die Menge der Immunzellen, insbesondere der aktivierten Immunzellen, in der Haut, die mit Invasomen enthaltend Immunsuppressivum behandelte wurde, gegenüber der Haut, die mit Invasomen ohne Immunsuppressivum behandelte wurde, um mindestens ca. 30%, vorzugsweise um mindestens ca. 100%, bevorzugter um mindestens ca. 200% und am meisten bevorzugt um mindestens ca. 500% verringert ist. Bei der Verwendung eines Immunstimulanz als pharmakologischer Wirkstoff liegt eine therapeutische Wirkung vor, wenn die Expression des jeweils untersuchten Zytokins in der Haut, die mit Invasomen enthaltend ein Immunstimulanz behandelte wurde, gegenüber der Haut, die mit Invasomen ohne Immunstimulanz behandelte wurde, um mindestens das ca. 2-fache, vorzugsweise um mindestens das ca. 5-fache, bevorzugter um mindestens das ca. 10-fache und am meisten bevorzugt um mindestens das ca. 20-fache erhöht ist oder wenn die Menge der Immunzellen, insbesondere der aktivierten Immunzellen, in der Haut, die mit Invasomen enthaltend Immunstimulanz behandelte wurde, gegenüber der Haut, die mit Invasomen ohne Immunstimulanz behandelte wurde, um mindestens ca. 30%, vorzugsweise um mindestens ca. 100%, bevorzugter um mindestens ca. 200% und am meisten bevorzugt um mindestens ca. 500% erhöht ist. Geeignete Zytokine, deren Konzentration zur Bestimmun der pharmazeutischen Wirkung untersucht werden können umfassen beispielsweise Interferon-alpha, Interferon-beta, Interferon-gamma, Interleukin-2, Interleukin-7, Interleukin-10, Interleukin-12, Interleukin-18, GM-CSF, TGF-beta und/oder TNF-alpha.. Die Anzahl der Immunzellen in den verschiedenen Regionen der Haut lässt sich beispielsweise durch immunhistologische Untersuchung von Dünnschnitten der Haut oder durch FACS-Analyse von Einzelzellsuspensionen der Haut, die zuvor mit einem immunzellspezifischen Marker inkubiert wurden, bestimmen. Geeignete Marker sind beispielsweise anti-CD28, anti-DC3, anti-CD86 und/oder anti-MHC II Antikörper.

Bei den weiteren pharmakologischen Wirkstoffen kann von einer therapeutischen Wirkung dann ausgegangen werden, wenn die jeweiligen Krankheitssymptome innerhalb eines Zeitraums von 10-200 Tagen der Anwendung deutlich gebessert werden. Eine deutliche Besserung liegt dann vor, wenn die von einem Mediziner bestimmbaren Krankheitsparameter, wie beispielsweise die Größe einer Läsion, die Stärke der Rötung, das Wachstum der Tumorzellen, um mindestens ca. 30%, vorzugsweise um 50%, noch bevorzugter um ca. 80%, am bevorzugtesten um ca. 100% zurückgeht.

Das Immunsystem der Haut bilden zum einen die in der Haut bereits vorhandenen Zellen, wie beispielsweise dendritische Zellen, aber auch die jeweils bei Erkrankung, Infektion und/oder entzündlichen Prozessen in die Haut infiltrierenden Immunzellen, wie beispielweise T-Zellen, Langerhans-Zellen und Makrophagen. Bei einer Beteiligung des Immunsystems der Haut ist beispielsweise eine gegenüber gesunder Haut erhöhte Infiltration der Immunzellen in die Haut bzw. Aktivierung der genannten Immunzellen zu beobachten, ohne dass tatsächlich eine Verwundung oder Infektion der Haut vorliegt. Ob an einer Hauterkrankung das Immunsystem der Haut beteiligt ist, lässt sich durch die klinisch bestimmbaren Parameter Rötung, Erwärmung und/oder Schwellung der Haut erkennen. Bei Vorliegen dieses Befunds wird vorzugsweise als weiteres Kriterium die Menge der in der Haut exprimierten Zytokine und/oder die Anzahl der Immunzellen bzw. aktivierten Immunzellen in der Haut herangezogen.

In einer bevorzugten Ausführungsform des erfindungsgemäßen Invasoms enthält das Invasom kein Tensid, da Tenside zu einer Erhöhung der Permeationsfähigkeit von Liposomen führen, die zu einer vollständigen Durchdringung der Haut und damit zu einer systemischen Wirkung des pharmakologischen Wirkstoffsführen. Der Begriff Tenside umfasst im Rahmen dieser Erfindung nichtionische Tenside, wie beispielsweise Tween oder Triton, zwitterionische Tenside wie beispielsweise CHAPS oder CHAPSO, kationische oder anionische Tenside, wie beispielsweise Laurylsulfat oder Lauroleyl jedoch nicht Lysophosphatide, wie beispielsweise Lysophosphatidylcholin, Lysophosphatidylethanolamin Lysophosphatidylinositol, Monolysocardiolipin, Dilysocardiolipin oder Lysophosphatidylserin.

Ein weiterer Gegenstand der vorliegenden Erfindung ist ein Verfahren zur Herstellung eines Invasoms, dadurch gekennzeichnet, dass das Lipidgemisch, das ein oder mehrere Lipide und ein oder mehrerer Lysophosphatide enthält, wobei die Lysophosphatide an dem Lipidgemisch einen Anteil im Bereich von ca. 0,1 bis ca. 40 Gew.-% haben, und mindestens ein pharmakologischer Wirkstoff, insbesondere ein immunmodulierender Wirkstoff, gemischt werden.

In einer bevorzugten Ausführungsform des erfindungsgemäßen Verfahrens enthält das Lipidgemisch neutrale Lipide, anionische Lipide oder ein Gemisch beider Lipidformen, insbesondere jedoch neutrale Lipide, in einen Anteil im Bereich von ca. 40 bis höchstens ca. 99,9 Gew.-%.

In einer Ausführungsform des erfindungsgemäßen Verfahrens wird eine alkoholische Lösung des Lipidgemischs mit mindestens einem pharmakologischen Wirkstoff, insbesondere einem immunmodulierenden Wirkstoff, beispielsweise durch Vortexen gemischt. Zur Lösung des Lipidgemischs geeignete Alkohole sind beispielsweise Ethanol, n-Propanol, Isopropanol, n-Butanol, Isobutanol und/oder n-Pentanol aber auch längerkettige Alkohole oder höhere Alkohole, die geeignet sind das jeweilige Lipidgemisch zu lösen. Vor oder nach der Zugabe mindestens eines pharmakologischer Wirkstoff, insbesondere eines immunmodulierender Wirkstoffs, können dem Gemisch ggf. Terpene hinzugefügt werden. Das Lipidgemisch kann in dem Alkohol in einem Gewichtsverhältnis im Bereich von ca. 10:1 bis ca. 1:100 (Alkohol:Lipidgemisch), vorzugsweise im Bereich von ca. 2:1 bis ca. 1:20, bevorzugterweise im Bereich von ca. 1:1 bis ca. 1:10, noch bevorzugter von ca. 1:2 bis ca. 1:8 und am meisten bevorzugt von ca. 1:3 bis ca. 1:4 gelöst werden.

Das Gemisch (mit oder ohne Terpen(e)) kann anschließend durch Ultraschall sonifiziert werden. In einem weiteren Schritt kann dem Gemisch aus Lipidgemisch und einem pharmakologischen Wirkstoff, insbesondere einem immunmodulierende Wirkstoff, das ggf. Alkohol und/oder Terpene enthält, unter Rühren Puffer oder auch destilliertes Wasser hinzugefügt werden. Als Puffer ist jeder physiologisch verträgliche Puffer, insbesondere Phosphatpuffer, geeignet. Der zugesetzte Puffer oder das zugesetzte Wasser kann dabei in einem Bereich von ca. 5 bis ca. 98 Gew.-%, vorzugsweise von ca. 30 bis ca. 95 Gew-%, besonders bevorzugt von ca. 60 bis ca. 90 Gew-%, noch bevorzugter von ca. 80 bis ca. 88 Gew.-% an der Gesamtmischung ausmachen. Durch diesen Schritt entsteht eine sogenannte grobkörnige Suspension, in der die erfindungsgemäßen Invasomen aufgeschlämmt sind. Die so hergestellten Invasomen sind im Puffer suspendiert, können aber auch Puffer oder Wasser enthalten.

Diese multilamellaren Vesikel können zur Herstellung von Invasomen bestimmter Größe in einem weiteren Schritt beispielsweise sonifiziert werden oder durch Polycarbonatmembranen mit definierter Porenweite, wie beispielsweise 400, 200, 100 oder 50 nm, extrudiert werden.

Ein weiteres im Stand der Technik bekanntes Verfahren zur Herstellung von Liposomen, das auch zur Herstellung der erfindungsgemäßen Invasomen geeignet ist, ist das Rotationsverdampfungsverfahren (Weiner et al., Antimicrob. Agents Chemother. 33: 1217-1221, 1989). Bei diesem Verfahren wird das Lipidgemisch am Boden eines Rundkolbens getrocknet und dann durch das Hinzufügen einer wäßrigen Lösung, die die pharmakologischen Wirkstoffe, insbesondere immunmodulierende Wirkstoffe, und ggf. Terpene, Alkohole und/oder Puffer enthält, unter Schütteln dispergiert. Nach der Hydratation werden die so erhaltenen multilamellaren Vesikel beispielsweise durch Polycarbonatmembran definierter Porengröße extrudiert oder auch sonifiziert, um Invasomen einer bestimmten Größe zu erhalten.

In einer bevorzugten Ausführungsform des erfindungsgemäßen Verfahrens enthält das verwendete Lipidgemisch und der pharmakologische Wirkstoff, insbesondere immunmodulierende Wirkstoff, kein Tensid. Der Begriff Tenside umfasst im Rahmen dieser Erfindung nichtionische Tenside, wie beispielsweise Tween oder Triton, zwitterionische Tenside wie beispielsweise CHAPS oder CHAPSO, kationische oder anionische Tenside, wie beispielsweise Laurylsulfat oder Lauroleyl. Der Begriff Tensid umfasst jedoch nicht Lysophosphatide, wie beispielsweise Lysophosphatidylcholin, Lysophosphatidylethanolamin, Lysophosphatidylinositol, Monolysocardiolipin, Dilysocardiolipin oder Lysophosphatidylserin.

Die vorliegende Erfindung umfasst auch Invasomen, die nach einem der vorangenannten Verfahren hergestellt werden.

Ein weiterer Gegenstand der vorliegenden Erfindung ist ein Arzneimittel, das ein erfindungsgemäßes Invasom und geeignete Hilfs- und Zusatzstoffe enthält. Beispiele für solche Hilfs- und Zusatzstoffe sind physiologische Kochsalzlösungen, Ringer-Dextrose, Dextrose, Ringer-Laktat, entmineralisiertes Wasser, Stabilisatoren, Antioxidantien, Komplexbildner, antimikrobielle Verbindungen, Proteinaseinhibitoren, inerte Gase, Gelformulierungen, wie beispielsweise weiße Vaseline und/oder Paraffin. Das Arzneimittel kann für die topische Applikation auch in Form von Verbänden, Pflastern, Kompressen, Salben oder Gelen zubereitet sein.

Ein weiterer Gegenstand der vorliegenden Erfindung ist die Verwendung eines erfindungsgemäßen Invasoms zur Therapie einer Hauterkrankung. Hauterkrankungen, die mit den erfindungsgemäßen Invasomen therapiert werden können umfassen beispielsweise Entzündungen der Haut, Autoimmunerkerkrankungen der Haut, Allergien, Graft-versus-Host Reaktion, Mykosen, bakterielle bzw. virale Infektionen, Tumore und andere proliferative Erkrankungen der Haut.

Eine bevorzugte Verwendung der erfindungsgemäßen Invasomen ist die Therapie von Hauterkrankungen, die durch eine Modulation des Immunsystems der Haut behandelbar sind. Der Begriff Therapie umfasst dabei sowohl die kurative oder palliative Behandlung bereits bestehender Erkrankungen als auch die Prävention von Erkrankungen.

Das Immunsystem der Haut bilden zum einen die in der Haut bereits vorhandenen Zellen, wie beispielsweise dendritische Zellen, aber auch die jeweils bei Erkrankung, Infektion und/oder entzündlichen Prozessen in die Haut infiltrierenden Immunzellen, wie beispielweise T-Zellen, Langerhans-Zellen und Makrophagen. Bei einer Erkrankung, die durch eine Modulation des Immunsystems der Haut behandelbar ist, ist beispielsweise eine gegenüber gesunder Haut erhöhte Infiltration der Immunzellen in die Haut bzw. Aktivierung der genannten Immunzellen zu beobachten, ohne dass tatsächlich eine Verwundung oder Infektion der Haut vorliegt. Ob eine Hauterkrankung durch eine Modulation des Immunsystems der Haut behandelbar ist, lässt sich durch die klinisch bestimmbaren Parameter Rötung, Erwärmung und/oder Schwellung der Haut erkennen. Bei Vorliegen dieses Befunds wird als weiteres Kriterium die Menge der in der Haut exprimierten Zytokine und/oder die Anzahl der Immunzellen bzw. aktivierten Immunzellen in der Haut herangezogen.

Zytokine, die bei Hauterkrankungen, die durch eine Modulation des Immunsystems der Haut behandelbar sind, verstärkt exprimiert werden, umfassen beispielsweise Interferon-γ, Il-2, IL-1β oder Il-12. Die Menge der in der Haut exprimierten Zytokine lässt sich auf Nukleinsäureebene beispielsweise durch RT-PCR, "RNA-se Protection Assays" oder "Nuclear Run Ons" und auf Proteinebene beispielsweise durch "Western-Blots" oder ELISA bestimmen. Dem Fachmann sind weitere Verfahren bekannt, die es erlauben das Expressionsniveau von Zytokinen in der Haut zu bestimmen. Hierbei wird das jeweils bestimmte Expressionsniveau in der erkrankten Haut mit dem Expressionsniveau in gesunder Haut verglichen. Eine Hauterkrankung, die durch eine Modulation des Immunsystems der Haut behandelbar ist, liegt vor, wenn die Expression des jeweiligen Zytokins in der kranken Haut gegenüber der gesunden Haut um mindestens das ca. 2-fache, vorzugsweise um mindestens das ca. 5-fache, erhöht ist. In diesem Fall wird als pharmakologischer Wirkstoff ein Immunsuppressor verwendet.

Eine Hauterkrankung, die durch eine Modulation des Immunsystems der Haut behandelbar ist, liegt auch dann vor, wenn die Expression des jeweiligen Zytokins in der kranken Haut gegenüber der gesunden Haut auf weniger als 50%, vorzugsweise auf weniger als 20% erniedrigt ist. In diesem Fall wird als pharmakologischer Wirkstoff ein Immunstimulanz verwendet.

Immunzellen, die bei Hauterkrankungen, die durch eine Modulation des Immunsystems der Haut behandelbar sind,, in größerer Menge in der Haut vorkommen, umfassen beispielsweise CD4⁺-Zellen, CD8⁺-Zellen, Langerhans-Zellen und Makrophagen. Die Menge der in der Haut vorkommenden Immunzellen lässt sich beispielsweise durch FACS-Analyse, histologisch und/oder immunhistologisch bestimmen. Dem Fachmann sind weitere Verfahren bekannt, die es erlauben, die Menge der Immunzellen in der Haut zu bestimmen. Hierbei wird die jeweils bestimmte Immunzellmenge, vorzugsweise die Menge der aktivierten Immunzellen, in der erkrankten Haut mit der Menge der Immunzellen, vorzugsweise der Menge der aktivierten Immunzellen, in gesunder Haut verglichen. Neben dem Kriterium der Zytokinexpression ist daher ein weiteres Kriterium das allein oder gleichzeitig mit dem vorangenannten Kriterium vorliegen kann die Menge der aktivierten Immunzellen in der Haut. Eine Hauterkrankung, die durch eine Modulation des Immunsystems der Haut behandelbar ist, liegt daher auch dann vor, wenn die Menge der Immunzellen, insbesondere der aktivierten Immunzellen, in der kranken Haut gegenüber der gesunden Haut um mindestens ca. 50%, vorzugsweise um mindestens ca. 100%, bevorzugter um mindestens ca. 200% und am meisten bevorzugt um mindestens ca. 500% erhöht ist. In diesem Fall wird als pharmakologischer Wirkstoff ein Immunsuppressor verwendet.

Eine Hauterkrankung, die durch eine Modulation des Immunsystems der Haut behandelbar ist, liegt auch dann vor, wenn die Menge der Immunzellen, insbesondere der aktivierten Immunzellen, in der kranken Haut gegenüber der gesunden Haut um mindestens ca. 50%, vorzugsweise um mindestens ca. 100%, bevorzugter um mindestens ca. 200% und am meisten bevorzugt um mindestens ca. 500% erniedrigt ist. In diesem Fall wird als pharmakologischer Wirkstoff ein Immunstimulanz verwendet.

Eine Hauterkrankung, die durch eine Modulation des Immunsystems der Haut behandelbar ist, liegt aber auch dann vor, wenn die Menge der Immunzellen in der kranken Haut gegenüber der gesunden Haut nicht wesentlich verändert ist, die erkrankten Zellen jedoch durch eine spezifische, durch ein Therapeutikum vermittelte Stimulierung des Immunsystems vom Organismus eliminiert werden können. Beispiele hiefür sind Tumoren oder andere proliferative Erkrankungen der Haut.

Hauterkrankungen, die durch eine Suppression des Immunsystems der Haut behandelbar sind, sind beispielsweise Alopecia areata, Alopecia totalis, Alopecia subtotalis, Alopecia universalis, Alopecia diffusa, atopische Dermatitis, Lupus erythematodes der Haut, Lichen planus, Dermatomyostis der Haut, atopisches Ekzem, Neurodermitis, Morphea, Sklerodermie, Psoriasis vulgaris, Psoriasis capitis, Psoriasis guttata, Psoriasis inversa, Alopecia areata Ophiasis-Typ, androgenetische Alopezie, allergisches Kontaktekzem, irritatives Kontaktekzem, Kontaktekzem, Pemphigus vulgaris, Pemphigus foliaceus, Pemphigus vegetans, vernarbendes Schleimhautpemphigoid, bullöses Pemphgoid, Schleimhautpemphigoid, Dermatitis, Dermatitis herpetiformis duhring, Urticaria, Necrobiosis lipoidica, Erythema nodosum, Lichen vidal, Prurigo simplex, Prurigo nodularis, Prurigo acuta, lineare IgA Dermatose, polymorphe Lichtdermatose, Erythema solaris, Lichen sclerosus et atrophicans, Exanthem der Haut, Arzneimittelexanthem, Purpura chronica progressiva, dihidrotisches Ekzem, Ekzem, fixes Arzneiexanthem, photoallergische Hautreaktion, Lichen simplex eriorale Dermatitis oder "Graft versus Host-Disease".

Eine besonders bevorzugte Verwendung der erfindungsgemäßen Invasomen ist die Prävention und/oder Therapie der Alopecia areata (alle klinischen Formen), der Psoriasis vulgaris (alle klinischen Formen), der atopischen Dermatitis, dem atopischen Ekzem, der Neurodermitis, der polymorphe Lichtdermatose, des Erythema solaris, des allergischen und irritativem Kontaktekzems, des Arzneimittelexanthems und/oder der "Graft versus Host-Disease"

Eine weitere Verwendung der erfindungsgemäßen Invasomen ist die Therapie einer Erkrankung, die durch eine Modulation des Immunsystems behandelbar ist. Eine Erkrankung, die durch eine Modulation des Immunsystems behandelbar ist, liegt dann vor, wenn die erkrankten Zellen durch eine spezifische, durch ein Therapeutikum vermittelte Stimulierung des Immunsystems vom Organismus eliminiert werden können. Krankheiten, die durch eine solche Modulation des Immunsystems behandelbar sind umfassen beispielsweise Tumore, Hyperplasien und/oder andere proliferative Erkrankungen, Arthritis, Viruserkrankungen sowie bakterielle und/oder parasitäre Infektionen..

Bei dieser Verwendung können die Invasomen auch in anderer geeigneter Weise, z.B. intraperitoneal oder intraartikulär, appliziert werden.

Eine weitere Verwendung der erfindungsgemäßen Invasomen ist die prophylaktische und therapeutische Vakzinierung bzw. Immuntherapie durch topische Applikation. Prophylaktisch bzw. therapeutische Vakzinierungen, die mit den erfindungsgemäßen Invasomen durchgeführt werden können, umfassen beispielsweise Tumorerkrankungen, Viruserkrankungen, sowie bakterielle und/oder parasitäre Infektionen.

Des weiteren zeigt Beispiel 8, dass selbst erfindungsgemäße Invasomen, die nicht mit einem pharmakologischen Wirkstoff beladen sind bereits zu einer Aktivierung von Zellen des Immunsystems, beispielsweise Langerhanschen-Zellen führen. Die erfindungsgemäßen Invasomen sind daher besonders für den Transport immunmodulierender pharmakologischer Wirkstoffe geeignet, da sie, quasi als Adjuvanz, die Wirkung von immunmodulierenden pharmakologischen Wirkstoffen verstärken können. Ein weiterer Gegenstand der vorliegenden Erfindung ist daher die Verwendung eines Invasoms bestehend aus einem Lipidgemisch, wobei das Lipidgemisch ein oder mehrere Lipide und ein oder mehrere Lysophosphatide in einem Anteil im Bereich von ca. 0,1 bis ca. 40 Gew.-% am Lipidgemisch enthält, als Adjuvanz bei der Behandlung von Erkrankungen, die durch eine Modulation des Immunsystems behandelbar sind, insbesondere Hauterkrankungen, die durch eine Modulation des Immunsystems der Haut behandelbar sind.

In einer bevorzugten Ausführungsform sind die Lipide neutrale, anionische oder ein Gemisch aus neutralen und anionischen Lipiden.

In einer bevorzugten Ausführungsform des erfindungsgemäßen Verwendung liegt der Gewichtsanteil der neutralen, anionischen oder eines Gemisches aus neutralen oder anionischen Lipids, vorzugsweise eines neutralen Lipids, an dem Lipidgemisch im Bereich von ca. 40 bis ca. 99.9 Gew.-%, vorzugsweise von ca. 50 bis ca. 98 Gew.-%, bevorzugter im Bereich von ca. 60 bis ca. 95 Gew.%, noch bevorzugter im Bereich von ca. 75 bis ca. 95 Gew.-% und am meisten bevorzugt ca. 90 Gew.-%.

In einer bevorzugten Ausführungsform der erfindungsgemäßen Verwendung enthalten die Invasomen die Lysophosphatide Lysophosphatidylcholin, Lysophosphatidylethanolamin Lysophosphatidylinositol, Monolysocardiolipin, Dilysocardiolipin oder Lysophosphatidylserin, insbesondere Lysophosphatidyl-cholin. Dabei liegt in einer bevorzugten Ausführungsform der erfindungsgemäßen Verwendung der Anteil der Lysophosphatide an dem Lipidgemisch im Bereich von ca. 1 bis ca. 25 Gew.-%, vorzugsweise von ca. 4 bis ca. 15 Gew.-% und am bevorzugtesten von ca. 10 bis ca. 15 Gew.-%.

Wenn die Invasomen als Adjuvanz verwendet werden können diese entweder einen pharmakologischen Wirkstoff einschließen und/oder der pharmakologischen Wirkstoff kann zu den bereits gebildeten Invasomen hinzugefügt werden. So ist es möglich einen ersten pharmakologischen Wirkstoff in das Invasom einzuschließen und einen zweiten der Invasomensuspension hinzuzufügen.

Die folgenden Beispiele und die Tabelle dienen lediglich der Illustration des Erfindungsgedankens, schränken jedoch den Gegenstand der Erfindung nicht ein.

Für den Fachmann ist es offensichtlich, dass eine Vielzahl von Modifikationen und Variationen der Zusammensetzungen und der Verfahren dieser Erfindung durchgeführt werden können. Es ist daher beabsichtigt, dass diese Erfindung solche Modifikationen und Variationen unter der Voraussetzung umfasst, dass sie im Schutzumfang der Ansprüche und deren Äquivalente liegen.

Die Prioritätsanmeldung DE 10105659.1, eingereicht am 8. Februar 2001, und alle hierin zitierten Dokument werden durch Verweis in die Beschreibung aufgenommen.

Die folgenden Beispiele sollen die Erfindung nur näher beschreiben ohne sie zu beschränken.

### Figuren

- Figur 1:: Größe von Invasomen, die unter identischen Bedingungen hergestellt wurden, in Abhängigkeit von der zugesetzten Terpenmenge.
- Figur 2:: Anisotropie von Invasomen, die unter identischen Bedingungen hergestellt wurden, in Abhängigkeit von der zugesetzten Terpenmenge.

- Figur 3:: Anreicherung von Cyclosporin A im Stratum corneum der Haut 6 h nach nicht okklusiver Behandlung mit Cyclosporin A-haltigen Invasomen.
- Figur 4:: Profil der Verteilung von ³H-Cyclosporin A in abdominaler menschlicher Haut 6 Stunden nach nicht okklusiver Behandlung mit Cyclosporin A-haltigen Liposomen bzw. Invasomen (30 µl/cm²). Die aufgetragene Invasomenmischung hatte einen Lipidgehal von 10% w/w.
- Figur 5:: FACS-Analyse von Einzelzellsuspensionen aus Epidermis, auf die zuvor topisch FITC-enthaltende leere Invasomen aufgetragen wurden.
- Figur 6:: Epidermales Häutchen ("sheet") nach topischer Applikation von leeren Invasomen. Die dendritischen Zellen sind immunhistochemisch mittels Peroxidasetechnik dunkel hervorgehoben.

### Tabellen

- Tabelle 1:: Haarwachstum nach Behandlung mit Invasomen, die Cyclosporin A oder Cyclosporin A und Terpene enthalten, im DEBR-Rattenmodell. I: Invasomen mit Terpenen (D-Limonen, Citral und Cineol); F: Invasomen ohne Terpene; C: Kontrolle. Als Kontrollgruppe wurden Ratten mit natürlichem Haarwachstum gewählt. Das Haarwachstum wurde anhand der folgenden frei gewählten Skala beurteilt: - = große Regionen der Haut sind völlig haarfrei; + = Bereiche der Haut mit sehr spärlichem Haarbewuchs; ++ = Bereiche der Haupt mit mittlerem Haarwachstum; +++ = scheinbar normale Haardichte.
- Tabelle 2:: Die bei Verwendung verschiedener Liposomen bzw. Invasomen in den einzelnen Hautschichten akkumulierte Menge Cyclosporin A ist in % der aufgetragenen Cyclosporin A Menge angegeben. Die Gesamtmenge des in und auf der Haut bestimmten Cyclosporins ist in der letzten Zeile angegeben. Die aufgetragene Invasomenmischung hatte einen Lipidgehalt von 10% w/w.
- Tabelle 3:: Die 48 h nach Verwendung von Cyclosporin A haltigen Invasomen in den Organen Leber, Milz, Nieren akkumulierten Cyclosporin A Mengen wurden bei drei Ratten bestimmt.
- Tabelle 4:: Anzahl der CD86-positiven Zellen im mikroskopischen Sichtfeld bei topischer Behandlung mit leeren Invasomen und mit FITC-beladenen Invasomen.

### Beispiele

### 1. Herstellung der Invasomen

Die Invasomen wurden mit dem Alkohol-Lösungsverfahren hergestellt. Eine geeignete Menge Phospholipon 80 (Nattermann GmbH, Deutschland) und Ethanol (im Verhältnis 3:1) wurden gemischt und für 5 Minuten gevortext. Dann wurde Cyclosporin A (0,5 Gewichts-% an der Endmischung) und D-Limonen, Cineol und Citral (im Verhältnis 10:45:45 v/v; 2 Gewichts-% an der Endmischung) hinzugefügt und das Gemisch erneut gevortext. Daran schloss sich für 5 Minuten eine Sonifizierung mit Ultraschall an (Bransonic ultrasonic cleaner, Connecticut, USA). Eine geeignete Menge Phosphatpuffer pH 7,4 (86,17 Gewichts-% an der Endmischung im Falle, dass keine Terpene zugesetzt wurden oder 84,17 Gewicht-% an der Endmischung beim Zusatz von Terpenen) wurde tropfenweise unter fortgesetztem Rühren zu dem ethanolischen Gemisch aus Lipid und Cyclosporin hinzugesetzt, bis eine grobkörnige Suspension erhalten wurde. Diese multilammellaren Vesikel wurden dann sonifiziert oder mit einer Avestinpresse (Avestin EmulsiFlex- C5, Canada) zuerst für 5 min über einen 200 nm Polycarbonatfilter, dann für 10 min über einen 100 nm Polycarbonatfilter und schließlich über einen 50 nm Polycarbonatfilter filtriert, bis die Durchschnittsgröße der Invasomen 50-70 nm betrug. Alle Filter wurden von Costar (Costar Nuclepore Polycarbonate, USA) erhalten. Der Durchmesser der Invasomen wurde mit einem Zetasizer IV (Malvern Instruments, Malvern UK) bestimmt. Der Durchmesser der Invasomen war im Bereich von 50-70 nm. Anschließend wurde der Polydispersitätsindex (PI) als ein Maß für die Homogenität der erhaltenen Invasomenzubereitung beurteilt. Der PI für die Invasomen lag unter 0,3. Dieses Ergebnis deutet auf eine homogene Invasomenpopulation hin.

### 2. Behandlung von DEBR-Ratten mit Invasomen

15 DEBR-Ratten mit einem Durchschnittsalter von 19 Monaten (das Alter lag im Bereich von 14-26 Monate) und mit einem Gewicht im Bereich von 200-350 g wurden in dem Experiment verwendet. Die Ratten wurden in Einzelkäfigen mit Wasser und Nahrung nach Wunsch gehalten. Der beobachtete Haarverlust reichte von großen kahlen Flächen an den Flanken der Tiere und haarlosen Bereichen auf dem Kopf bis zum fast vollständigen Verlust aller Kopf- und Körperhaare. Die Ratten wurden in 3 Gruppen à 5 Tiere mit ähnlichem Haarverlust (Gruppe I, II und III) eingeteilt. Die Experimente wurden mit den beiden vorangehend beschriebenen Invasomentypen mit oder ohne zugesetzte Terpene durchgeführt. Die Invasomenkontrolle wurde in gleicher Weise, jedoch ohne Cyclosporin A hergestellt. Die liposomalen Zubereitungen wurden nicht-okklusiv auf dem Rücken (dorsale Seite) der Ratten aufgetragen. Für die Auftragung wurde ein abgegrenzter Bereich von 2 x 2 cm auf dem Rücken der Ratten markiert.

Die Gruppe I wurde verwendet, um die Wirksamkeit Cyclosporin beladener Invasomem, die D-Limonen, Citral und Cineol enthielten, auf das Haarwachstum zu untersuchen. Jeder Ratte wurde für 7 Wochen 2-mal am Tag 20-80 µl/cm² der Invasomenzubereitung auf einen markierten 2 x 2 cm großen Bereich an einer der kahlen Flanken aufgetragen, während die gegenüberliegende (kontralaterale) Flanke mit der Kontrollzubereitung behandelt wurde.

Die Gruppe II wurde verwendet, um die Wirksamkeit Cyclosporin beladener Invasomen auf das Haarwachstum zu untersuchen. Jeder Ratte wurde für 7 Wochen 2-mal am Tag 20-80 µl/cm² der Invasomenzubereitung auf einen markierten 2 x 2 cm großen Bereich an einer der kahlen Flanken aufgetragen, während die gegenüberliegende (kontralaterale) Flanke mit der Kontrollzubereitung behandelt wurde.

Ratten der Gruppe 3 wurde für 7 Wochen 2-mal am Tag 20-80 µl/cm² einer ethanolischen Cyclosporin A-Lösung (0,5%) innerhalb des markierten 2 x 2 cm großen Bereichs auf einer der kahlen Flanken aufgetragen, während die kontralaterale Flanke unbehandelt blieb.

Die morphologischen Änderungen wurden täglich untersucht. Vor der Behandlung hatten alle Ratten in Gruppe I und II große kahle Flanken in abdominalen, dorsalen, Kopf- und Schulterbereichen. Alle 5 Ratten der Gruppe I zeigten 7 Tage nach Behandlungsbeginn Haarwachstum auf der behandelten Seite, während kein Haarwachstum auf dem behandelten Kontrollbereich zu beobachten war. Am 21. Tag nach Behandlungsbeginn zeigten 4 der 5 Ratten spärlichen bis mittleren Haarwuchs und nach 36 Tagen hatte das Fell auf den behandelten Stelle wieder die normale Dichte erreicht. Während sich das Haarwachstum an der behandelten Stelle fortsetzte, war an anderen Stellen der Haut weiterer Haarverlust zu beobachten. Dies spricht dafür, dass die Wirkung des Cyclosporin A nur lokal auf die Auftragungsstelle begrenzt war, und keine oder nur eine geringe systemische Wirkung des Cyclosporins auftrat. Dafür sprach auch, dass in einer HPLC-Analyse des Serums der Ratten 14 Stunden nach der ersten Behandlung, aber auch am Ende der Studie kein Cyclosporin A im Blut nachgewiesen werden konnte. Eine der 5 Ratten zeigte bereits nach 21 Tagen ein normales Haarwachstum am gesamten Körper, jedoch einen verstärkten Effekt an der behandelten Stelle. Diese Beobachtung lässt sich beispielsweise dadurch erklären, dass 2-3% der DEBR-Ratten auch ohne Behandlung ein spontanes Nachwachsen aller Haare zeigen.

Bei den 5 Ratten der Gruppe II wurden erste Anzeichen auf Haarwuchs bei allen Ratten 14 Tage nach Beginn der Behandlung beobachtet, während auf den Kontrollflächen kein Haarwachstum beobachtet werden konnte. Das Haarwachstum setzte sich innerhalb und unmittelbar angrenzend an die Anwendungsflächen fort und am 21. Tag war schwaches bis moderates Haarwachstum auf den behandelten Regionen zu beobachten. Das Haarwachstum setzte sich fort und führte nach etwa 42 Tagen an der Behandlungsstelle zur normalen Felldichte. 14 Stunden nach Beginn der Behandlung und am Ende der Behandlung konnte mit HPLC-Analyse keine Cyclosporin A im Blut der Ratten nachgewiesen werden. Wiederum zeigte eine der Ratten, wie bereits bei den Ratten der Gruppe I beobachtet, Haarwachstum am gesamten Körper.

Die Ratten der Gruppe III zeigten während des gesamten Behandlungszeitraums kein Haarwachstum, vielmehr setzte sich der Haarverlust an anderen Stellen des Körpers bei allen Ratten in dieser Gruppe fort.

Die vorangehend beschriebenen Ergebnisse sind in der Tabelle 1 zusammengefasst.

### 3. HPLC-Analyse

Das verwendete HPLC-System war eine Merck-Hitachi HPLC 655 A-12 Flüssigchromatographiepumpe, die mit einem Kontron 360 Autosampler und mit einer Merck-Hitachi L-5000 LC-Kontrolleinheit ausgerüstet war. Die verwendete Säule war eine LiChrospher 100 RP-18-Säule, die sphärisch geformte 5 µm Silicagelpartikel enthielt. (3 mm I.D. und 125 mm Länge). Der UV-Detektor war ein Merck-Hitachi L-4000 UV-Absorptionsdetektor. Der verwendete Integrator war ein Merck-Hitachi D-7500 Integrator. Die für die Elution verwendete Flüssigphase war Methanol:Wasser:Acetonitril im Verhältnis 10:30:60. Die Flussrate betrug 0,7 ml/min, wobei die Säulentemperatur auf 75°C gehalten wurde. Der verwendete Druck betrug 43-44 bar. Die UV-Detektion erfolgte bei 208 nm. Die Retentionszeit für Cyclosporin A war 8,65 Minuten.

Eine Cyclosporin-Standardlösung wurde zur Eichung des Systems verwendet, wobei das Detektionslimit bei ca. 50 ng/ml lag. Über den gesamten Eichbereich bis 7000 ng/ml Cyclosporin A wurde ein linearer Zusammenhang zwischen Absorption und Konzentration beobachtet.

### 4. Einfluss von Terpenen auf die Membran von Invasomen

Die Invasomen wurden wie unter 1. beschrieben hergestellt statt 0,5 Gew.-% wurde jedoch nur 0,35 Gew.-% Cyclosporin A verwendet. Von dem Terpengemisch bestehend aus 45% Cineol, 45% Citral und 10% D-Limonen wurden 0, 0,25, 0,5, 0,75, 1,0, 1,25, 1,5 oder 2 Gew.-% hinzugesetzt. Entsprechend der zugesetzten Menge Terpen und Cyclosporin A wurde phosphatgepufferte Kochsalzlösung (PBS) bis 100 Gew.% zugesetzt. Anschließend wurde die Suspension für je 30 min im Emulsiflex C5 sukzessiv durch 400 nm, 200 nm, 100 nm und 50 nm Polycarbonat-Membranen bei offenem Homogenisatorspalt extrudiert. Der Durchmesser der Invasomen wurde mit einem Zetasizer IV bestimmt.

Die Größe der so hergestellten Invasomen, die 2% Terpengemisch enthielten, betrug:

| | |
|---|---|
| Invasomen | 87,6 ± 0,5 nm |
| Invasomen mit Terpenen | 85,1 ± 0,4 nm |
| Cyclosporin-Invasomen | 74,1 ± 0,8 nm |
| Cyclosporin-Invasomen mit Terpenen | 108,4 ± 0,9 nm |

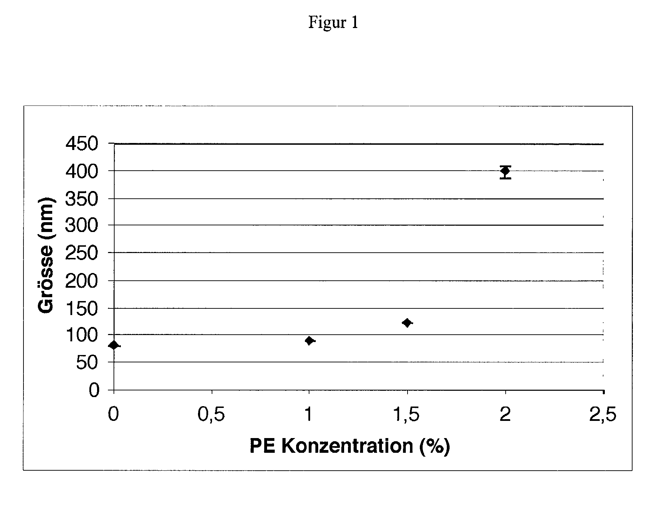

Die Invasomen wurden wie oben beschrieben hergestellt. Alle eingesetzten Mengen/Volumina wurden auf 1/10 reduziert. Die Emulsionen wurden mit einen LiposoFast der Firma Avestin sukzessiv durch einen 400 nm, 200 nm, 100 nm und 50 nm Filter je 21 Mal extrudiert. Die Konzentration der Terpene wurde wie oben beschrieben variiert. Die Größe der Invasomen in Abhängigkeit von Gewichtsanteil des Terpengemischs ist in Figur 1 dargestellt.

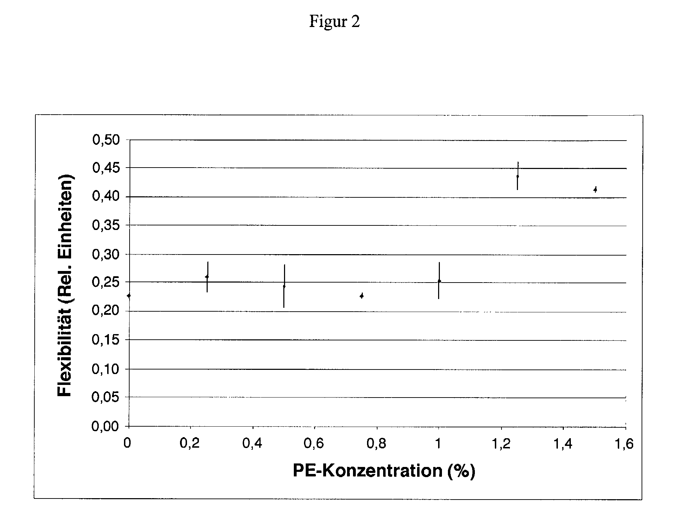

In einem weiteren Experiment wurde die Invasomenmembran mit dem Fluorophor DPH (Diphenylhexatrien) in einem molaren Verhältnis von 1:400 (DPH/Lipid) markiert und die Anisotropie der Membran in Abhängigkeit von der Terpen-Konzentration fluoreszenzspektroskopisch bestimmt. Das Ergebnis ist in Figur 2 dargestellt.

Cyclosporin A-Invasomen ohne Terpengemisch hatten nach manueller Extrusion eine Größe von 79,9 ± 0,56 nm. Mit steigender Terpen-Konzentration nahm die Größe der Invasomen nach manueller Extrusion zu (Figur 1). Sie betrug bei 2 % Terpen und Extrusion durch einen 50 nm Filter 398,6 ± 10,8 nm. Das zeigt, dass PV zu einer Flexibilisierung der Membran führt.

Die Anisotropie, ein Maß für die Fluidität der Membran, nahm bei Konzentrationen oberhalb von 1 Gew.-% Terpengemisch von 0,25 rel. Einheiten auf über 0,4 rel. Einheiten zu (Figur 2). Das bedeutet, dass die Terpene die Membran flexibler machen.

### 5. Penetration von Cyclosporin A-Invasomen in die humane Haut ex vivo

Die Invasomen wurden wie unter 4. beschrieben hergestellt, wobei 25 µl fluoreszenzmarkiertes Cyclosporin A (2 mmol in DMSO) und unmarkiertes Cyclosporin A verwendet wurde. Einer Invasomen-Präparation wurde 1 Gew.% Terpengemisch zugesetzt. Die Lösung wurde mit 0,5 ml PBS (entsprechend etwa 83 Gew.-%) unter Schütteln (Vortex) hydratisiert. Anschließend wurden die Suspensionen mit einem Handextruder (LiposoFast, Avestin) durch die Filterkaskade von 400-50 nm extrudiert. Die Lipidendkonzentration betrug 10% und die Cyclosporin-Konzentration betrug ca. 0,35%. Die Invasomen oder eine ethanolische Lösung fluoreszenzmarkierten Cyclosporins als Kontrolle wurden für 6 h nicht-okklusiv auf humane abdominale Haut appliziert. Die Hautproben wurden in Tissue Tec eingebettet und 7 µm dicke Querschnitte mit einem Cryo-cut (-25 °C) durch die Haut angefertigt. Diese wurden im konfokalen Mikroskop bei maximaler Laserleistung, offenem Pin-hole und konstant gehaltenem Gain und Offset analysiert.

Fluoreszenzmarkiertes Cyclosporin, das in ethanolischer Lösung appliziert wurde, ist nur in den obersten Schichten der Epidermis (Stratum corneum) lokalisiert. Fluoreszenzmarkiertes Cyclosporin in Invasomen ohne Terpengemisch ist in der Epidermis und in geringerer Konzentrationen in der Dermis nachweisbar. Auch hier sind auf/in dem Stratum corneum Aggregate fluoreszenzmarkierten Cyclosporins sichtbar. Invasomen mit 1% Terpengemisch und fluoreszenzmarkiertem Cyclosporin A sind homogen im Stratum corneum verteilt und fluoreszierendes Cyclosporin A ist in der Dermis nachweisbar. Das bedeutet, dass das Terpengemisch in einer Konzentration von 1 % zu einer homogenen Verteilung von Cyclosporin A in der Epidermis führt und die Penetration in die tieferen Hautschichten verstärkt.

### 6. Der Einfluß von Lyso-Phosphatidylcholin auf die Penetration von Invasomen in humane Haut ex vivo

1,75 mg Cyclosporin A, 66,6 mg ethanolische Lipidlösung und 10 µCi ³H-Cyclosporin (10 µl ethanolische Lösung mit einer spez. Aktivität von 296 GBq/mmol) wurden in einem Rundkolben eingewogen. Dabei wurden die folgenden Lipide bzw. Lipidgemische verwendet:
100 % Soja-PC (99% Reinheit)
94 % Soja-PC + 6% Lyso-PC
90 % Soja-PC + 10 % Lyso-PC
85 % Soja-PC + 15 % Lyso-PC
100 % Phospholipon 80 (Soja-Lipid mit ca. 75% PC, ca. 5% Lyso-PC und ca. 20% andere Lipide)
100 % Phospholipon 100 (aufgereinigtes Soja-Lipid, das nur Phosphatidylcholin und Lysolecithin enthält)

Soja-Phosphatidylcholin (Soja-PC) und Lysophosphatidylcholin (Lyso-PC) wurden von Sigma-Aldrich (Seelze) und Phospholipon 80 (PL 80) und Phospholipon 100 (PL 100) von Nattermann bezogen. Wenn die Lipide in Chloroform gelöst vorlagen, wurde das Chloroform abrotiert und der Lipidfilm getrocknet. Durch Zugabe von Ethanol wurde eine ethanolische Lipidlösung hergestellt. Die Mischung wurde 5 min gevortext und 20 min im Ultraschallbad beschallt, bis das Cyclosporin vollständig gelöst war. Danach wurde die Lösung durch langsames Zugeben von 0,431 ml PBS unter vortexen hydratisiert und bei Raumtemperatur 30 min im Dunkeln inkubiert. Die erhaltenen Suspension wurde je 21 mal durch Polycarbonatfilter mit einem Porendurchmesser von 400, 200, 100 und 50 nm extrudiert. Es wurden 30 µl pro cm² dieser Emulsion nicht-okklusiv auf humane abdominale Haut aufgetragen, die in einer Franz Diffusionszelle eingespannt war, und über 6 h inkubiert. Alle Ansätze wurden 3 - 6-mal wiederholt. Anschließend wurde die Oberfläche der Haut mit Ethanol abgewischt und mit 20 Tesafilm-Abrissen die Hornschicht der Epidermis entfernt. Über das Gewicht des Tesafilm-Abrisses wurde die Dicke des Stratum corneum Abrisses berechnet. Anschließend wurde die Epidermis in 30 µm dicke Schichten geschnitten und die einzelnen Proben in Methanol/Essigsäure übernacht extrahiert. Nach Zugabe von RiaLuma wurde die Radioaktivität der Proben über 40 min bzw. mit einer Fehlertoleranz ≤ 2% bestimmt.

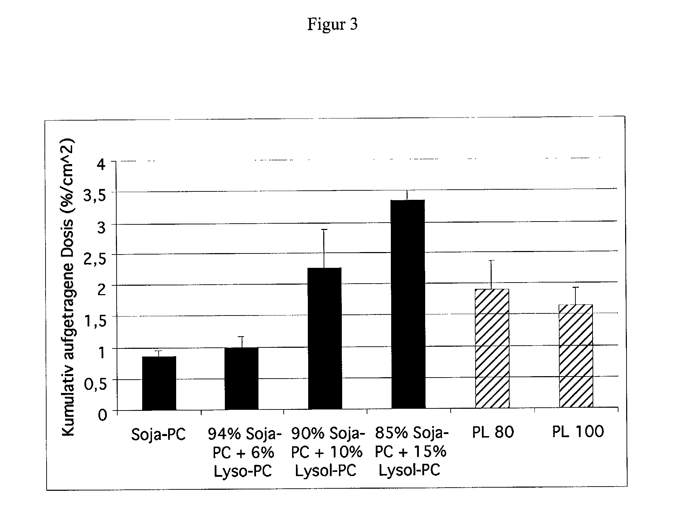

Mit steigender Lyso-PC Konzentration in Liposomen aus Soja-Lipid nimmt die Penetration des Cyclosporins in das Stratum corneum zu (Figur 3). Während 6 h nach der Applikation von reinem Soja-Lipid Liposomen nur 0,85 ± 0,11% des aufgetragen Cyclosporins im Stratum corneum sind, nimmt die Menge auf 3,35 ± 0,42% zu, wenn 15% Lyso-PC in den Liposomen vorhanden ist. Cyclosporin-Invasomen aus dem natürlichen Sojalipid-Extrakt Phospholipon 80 transportieren 1,92 ± 0,44% der aufgetragen Dosis in das Stratum corneum und Invasomen aus dem natürlichen Soja-Lipid Phospholipon 100, das nur aus Phosphatidylcholin und Lysolecithin besteht, transportieren 1,65 ± 0,27% in das Stratum corneum. In diesen natürlichen Extrakten beträgt das Verhältnis von Lysophosphatidylcholin zu Phosphatidylcholin 1:10. Liposomen aus 90% Phosphatidylcholin und 10% Lysophosphatidylcholin, penetrieren genauso gut oder besser (2,27 ± 0,10%) in das Stratum corneum wie Liposomen aus den natürlichen Extrakten Phospholipon 80 und Phospholipon 100.

Daraus folgt, dass Lysophosphatidylcholin die Penetration der erfindungsgemäßen Invasomen in die Haut verbessert.

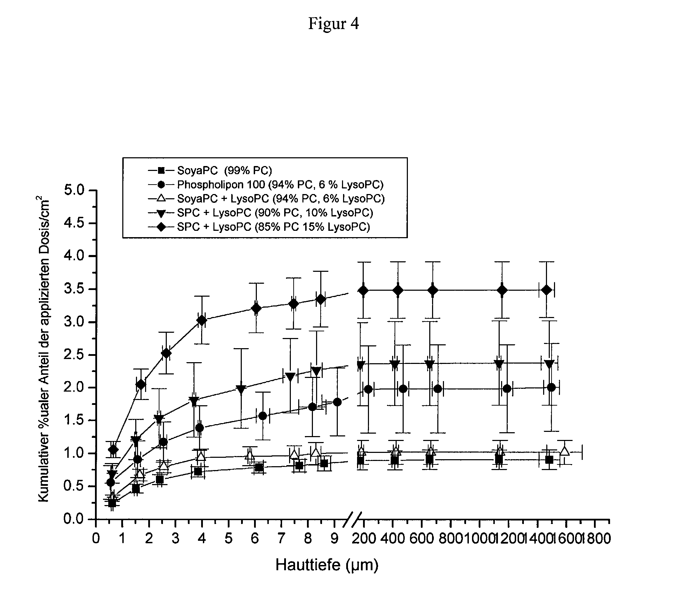

Das gleiche Bild erhält man, wenn man die kumulative Menge des topisch applizierten Cyclosporin A in den einzelnen Hautschichten bestimmt (Figur 4). Es zeigt sich auch hier, dass Liposomen mit 15% Lyso-Phosphatidylcholin am besten in die Epidermis penetrieren. Mit sinkender Lyso-PC Konzentration nimmt die Penetration ab. Invasomen aus den natürlichen Extrakten Phospholipon 80 oder Phospholipon 100 erhöhen die Penetration von Cyclosporin A in die Epidermis im gleichen Maß wie Liposomen aus Soja-PC mit einem Zusatz von 10 % Lyso-PC.

Tabelle 2 zeigt zusammenfassend die in den einzelnen Hautschichten akkumulierten Mengen an Cyclosporin A. Im Empfängerkompartiment (Receiver Compartment) unterhalb der Haut ist Cyclosporin A nur in Spuren nachweisbar. Dies zeigt, dass Cyclosporin A nicht in signifikantem Ausmaß durch die Haut hindurch penetriert. Das gesamte topisch applizierte Cyclosporin A wurde wieder gefunden (siehe letzte Spalte).

### 7. Penetration von Cyclosporin A-Invasomen durch die Haut im Tiermodell

### Quantifizierung von ³H-markiertem Cyclosporin A im Blut und Organen nach topischer Applikation von Invasporin

Drei DDAB Ratten wurden auf einer Fläche von 4 cm² mit 80 µl einer Suspension von Invasomen aus NAT-8539 (ethanolische Soja-Lipid Lösung mit einer Zusammensetzung wie Phopsholipon 80) enthaltend 3,5 mg/ml Cyclosporin und 0,5% Terpengemisch behandelt. Die applizierte Menge Cyclosporin A betrug 280 µg mit einer Gesamtaktivität von 9,6 µCi (spezifische Aktivität: 34,3 µCi pro mg Cyclosporin A). Bis zu zwei Tagen nach Applikation wurden zu verschiedenen Zeitpunkten (t = 0, 2h, 4h, 6h, 9h, 24h, 35h, 48h) je 100 µl Blut retroorbital entnommen. Nach 48 h wurden Leber, Milz und beide Nieren entnommen und Gewebeproben (ca. 200 mg) für die Cyclosporin A Bestimmung präpariert (Leber 4-fach, Milz und Nieren je 2-fach Bestimmung). Nach Aufarbeitung der Blut- und Gewebeproben (über Nacht Inkubation mit Perchlorsäure und Wasserstoffperoxid bei 60°C) wurde die ³H-Aktivität der Proben gemessen.

Die Blutproben aller drei Ratten enthielten zu keinem Zeitpunkt eine signifikante Menge an Radioaktivität. Alle Werte lagen deutlich unterhalb der Nachweisgrenze, die durch eine Eichkurven wie unter 3. bestimmt wurde und bei 4 ng/ml Cyclosporin A lag (0,4 ng pro 100 µl Blutprobe entspricht 30 dpm nach Abzug von 30 dpm Hintergrundaktivität).

Die Maximalwerte in den Organen (siehe Tabelle 3) betrugen für die Leber 38 ng/g Gewebe (Ratte 1: 323 ng/Gesamtorgan), für die Milz 12 ng/g Gewebe (Ratte 1: 13 ng/Gesamtorgan) und für die Nieren 14 ng/g Gewebe (Ratte 1: 25 ng in beiden Nieren). Dies entspricht einem prozentualen Anteil von maximal 0,13% der applizierten Dosis in diesen drei Organsystemen.

### 8. Aktivierung von Langerhanszellen durch leere Invasomen

### Anfertigung von epidermalen "sheets" und Immnunohistochemie:

Leere Invasomen, die wie unter 1 beschrieben jedoch ohne Cyclosporin A hergestellt wurden, wurden auf die Ohren (50 µl/Ohr) der narkotisierten Mäuse aufgetragen. Die Tiere wurden nach 16 h getötet und die Ohren abgetrennt. Zur Entfernung der Haare wurden die Ohren rasiert und dann entlang des Ohrknorpels mit Pinzetten auseinander gezogen, so dass zwei Ohrhälften (jeweils bestehend aus Dermis und Epidermis) entstanden. Die Ohrhälften wurden dann für 1 h bei 37°C in einer 20 mM EDTA-Lösung inkubiert und anschließend die Epidermis von der Dermis abgestreift. Die Epidermis erschien dabei als dünnes Häutchen ("epidermal sheet"), das für 10 min in Aceton (-20°C) fixiert wurde. Danach wurden die Langerhanszellen (LC) immunhistochemisch mittels Peroxidasetechnik dargestellt. LCs wurden mittels anti-MHC-II-Antikörper detektiert, da eine starke MHC-II-Expression charakteristisch ist für alle LCs und diese durch Aktivierung nur in geringerem Umfang moduliert wird. Zur Darstellung einer LC-Aktivierung wurde ein anti-CD86 Antikörper verwendet, da dieses Oberflächenantigen im Zuge der LC-Aktivierung deutlich (ca. 100-fach) hoch reguliert wird. Abschließend wurden die epidermalen sheets auf Objektträger aufgezogen, eine Kernfärbung durchgeführt und mikroskopisch ausgewertet.

### Herstellung einer Einzelzellsuspension (EZS) aus Mausohren:

Ohrhälften wurden wie oben beschrieben präpariert. Je zwei Ohrhälften wurden dann in 1 ml einer Enzymlösung (Collagenase III, Dispase, DNase; 0,4 mg/ml; 0,2 mg/ml; 0,16 mg/ml) für 30 min bei 37°C unter gelegentlichem Schütteln inkubiert, anschließend die Epidermis von der Dermis abgestreift und durch kräftiges Auf- und Abpiptettieren eine Einzelzellsuspension hergestellt. Die Zellzahl aus einem Ohr lag dabei zwischen 3 und 7 x 10⁵ Zellen. Das sind genügend Zellen für bis zu 4 verschiedene (Doppel-) Färbungen zur FACS-Analyse.

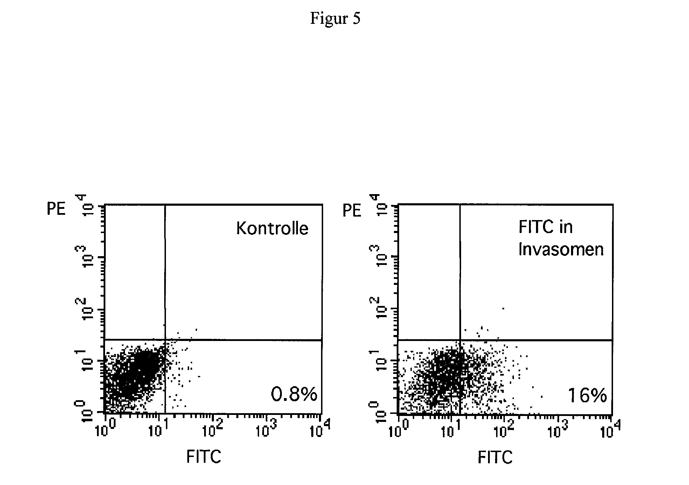

Invasomen (formuliert aus Phospholipon 80) mit enkapsuliertem Fluorezeinisothiocanat (FITC; Sigma-Aldrich; Seelze) wurden wie unter 1. beschrieben hergestellt. Es wurde jedoch statt 0,5 Gew.-% Cyclosporin A 0,35 Gew.-% FITC verwendet. FITC-Invasomen und eine ethanolische FITC-Lösung zum Vergleich wurden topisch auf Mausohren appliziert. 16 Stunden später wurden sowohl epidermale sheets als auch Einzelzellsuspensionen wie oben beschriebenen hergestellt und die Aufnahme von FITC in epidermale Zellen analysiert. Nach Applikation von FITC-Invasomen waren 16% der Zellen FITC-markiert (siehe die FACS-Analye in Figur 5), wohingegen nicht in Invasomen enkapsuliertes FITC nicht in signifikantem Ausmaß in die Haut eindringen kann. Dies lässt eindeutig auf eine effiziente Penetration der FITC-Invasomen in die Haut und deren Aufnahme bzw. ihrer Inhaltstoffe durch epidermale Zellen schließen

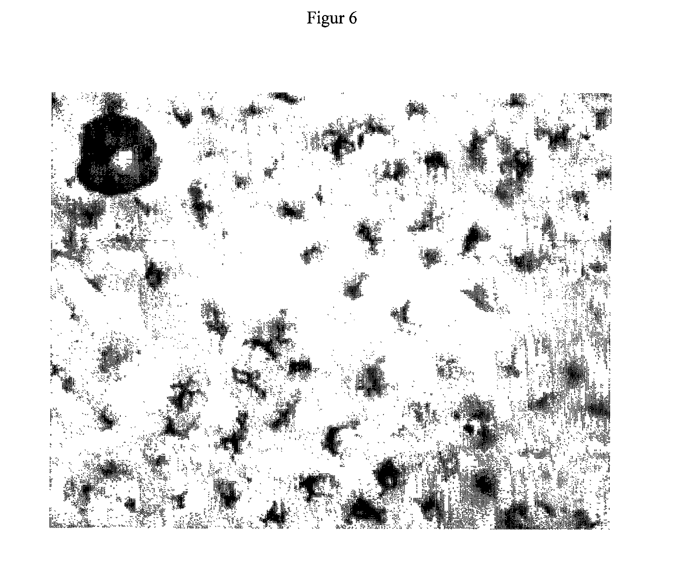

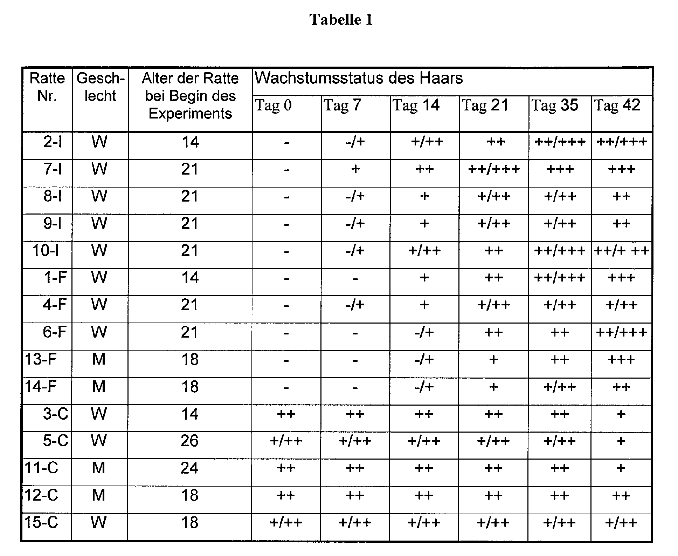

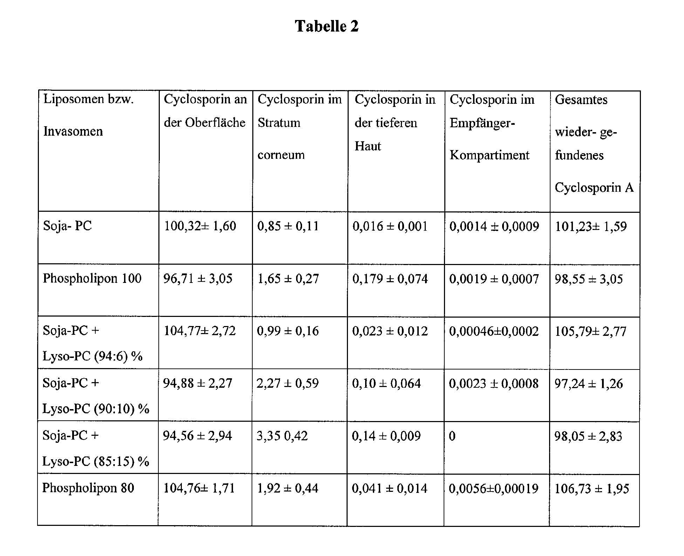

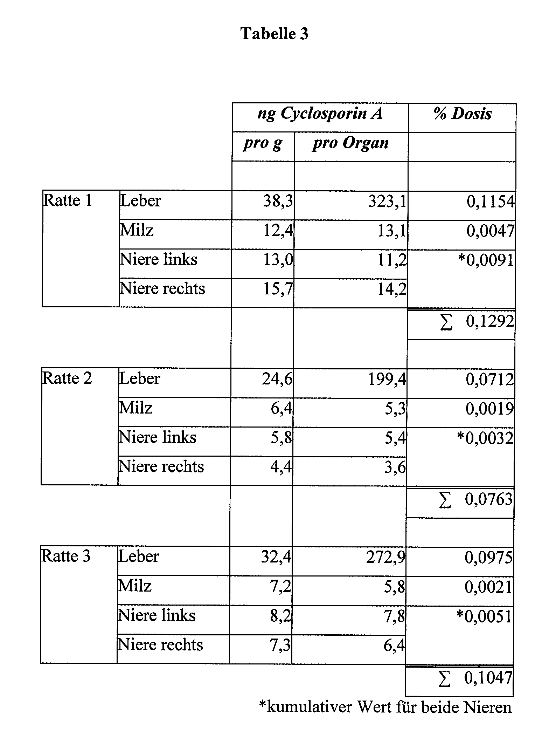

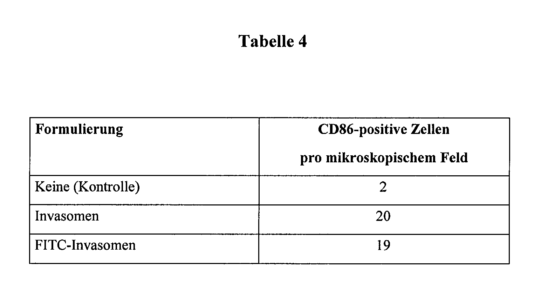

Leere Invasomen (formuliert aus Phospholipon 80) aktivierten LCs nach topischer Applikation in erheblichem Umfang, was durch die Hochregulation von CD86 in epidermalen sheets nachgewiesen werden konnte (Tabelle 4 und Figur 6).

## Patentansprüche

1. Invasom enthaltend:
a) ein Lipidgemisch umfassend ein oder mehrere Lipide und
ein oder mehrere Lysophosphatide, wobei die Lysophosphatide an dem Lipidgemisch einen Anteil im Bereich von 0,1 bis 40 Gew.-% haben; und
b) mindestens einen pharmakologischen Wirkstoff.

2. Invasom nach Anspruch 1, wobei die Lipide neutrale, anionische oder ein Gemisch von neutralen und anionischen Lipiden sind.

3. Invasom nach Anspruch 2, wobei die neutralen oder anionischen Lipide oder ein Gemisch von neutralen und anionischen Lipiden einen Anteil an dem Lipidgemisch im Bereich von 40 bis 99,9 Gew.-% haben.

4. Invasom nach einem der Ansprüche 1 bis 3, wobei das Invasom das Lipidgemisch und den pharmakologischen Wirkstoff in einem Gewichtsverhältnis von 1:1 bis 1000:1, vorzugsweise von 2:1 bis 100:1 enthält.

5. Invasom nach einem der Ansprüche 1 bis 4, wobei das Invasom einen Durchmesser von 30 bis 400 nm aufweist.

6. Invasom nach einem der Ansprüche 1 bis 5, wobei das Lipidgemisch aus Sojabohnen, Kattunsamen, Kokosnusskern, Erdnuss, Saflorsamen, Sesamsamen, Sonnenblumensamen, Leinsamen, Raps, Weizenkeimen, Oliven, Walfett, Stratum corneum Lipid, Rinderklauenöl und/oder Ei gewonnen wird.

7. Invasom nach einem der Ansprüche 3 bis 6, wobei die neutralen Lipide an dem Lipidgemisch einen Anteil im Bereich von 75 bis 95 Gew.-% haben.

8. Invasom nach einem der Ansprüche 2 bis 7, wobei die neutralen Lipide Glycerophospholipide, insbesondere Phosphatidylcholine, Steroide, Glycerophosphonolipide, Glycerophosphinolipide und/oder Sphingolipide sind.

9. Invasom nach einem der Ansprüche 1 bis 8, wobei die Lysophosphatide Lysophosphatidylcholine, Lysophosphatidylethanolamine, Lysophosphatidylinositol, Monolysocardiolipin, Dilysocardiolipin und/oder Lysophosphatidylserine sind.

10. Invasom nach einem der Ansprüche 1 bis 9, wobei das Invasom ein oder mehrere Terpene enthält.

11. Invasom nach Anspruch 10, wobei das Terpen Cineol, Citral, Limonen, insbesondere D-Limonen, Menthan, Terpinen, Terpinolen, Menthol, insbesondere 1-Menthol, Carveol, insbesondere 1-Carveol, Menthon, Carvon, Pinen, insbesondere β-Pinen, Caren, insbesondere 3-Caren, Terpineol, Terpinen-4-ol, Pulegon, Piperiton, Cyclohexanoxid, Limonenoxyd, Pinenoxyd, Cyclopentenoxid, Ascaridol, 7-Oxybicyclo-[2.2.1]-heptan, Cymol, Camphen, Citronellol, Geraniol, Nerol, Linalool, Borneol, Thujol, Sabinol, Myrtenol, Thymol, Verbenol, Fenchol, Piperitol, Perillaaldehyd, Phellandral, Citronellal, Myrtenal, Piperiton, Thujon, Umbellolun, Verbenon, Chrysanthenon, Fenchon, Campher, Chinon, Menthofuran, Linalooloxid, Rosenoxid und/oder Quinghaosu ist.

12. Invasom nach einem der Ansprüche 1 bis 11, wobei der pharmakologische Wirkstoff ein Immunsuppressivum, ein Immunstimulans, ein Antiallergikum, ein Antibiotikum, ein Antiinfektivum, ein Zytostatikum, ein Zytotoxikum, ein Mitogen, ein Chemokin, ein Cytokin, ein Dermatikum und/oder ein physiologischer oder pharmakologischer Inhibitor eines Mitogens, eines Chemokins oder eines Cytokins ist.

13. Invasom nach Anspruch 12, wobei das Immunsuppressivum ein Glucokorticoid, insbesondere Beclomethason, Betamethason, Clocortolon, Cloprednol, Cortison, Dexamethason, Fludrocortison, Fludroxycortid, Flumetason, Fluocinolonacetonid, Fluocinonid, Fluocortolon, Fluormetholon, Fluprednidenacetat, Hydrocortison, Paramethason, Prednisolon, Prednison, Prednylidin, Pregnenolon, Triamcinolon oder Triamcinolonacetonid, ein Cyclosporin, insbesondere Cyclosporin A, Mycophenolatmofetal, Tacrolimus, Rapamycin, FK 506, Cycloheximid-N-etylethanoat, Azathioprin, Ganciclovir, ein Anti-Lymphozytenglobulin, Ascomycin, Myriocin, pharmakologische Inhibitoren von MAP-Kinasen und/oder Methotrexat ist.

14. Invasom nach einem der Ansprüche 1 bis 13, wobei der pharmakologische Wirkstoff eine Nukleinsäure, ein Protein, ein Peptid, eine Zucker und/oder ein Lipid ist.

15. Invasom nach Anspruch 14, wobei die Nukleinsäure ein Antisense-Oligonucleotid, eine Antisense-RNA, eine RNAi, eine siRNA oder ein eine Triple-Helix ausbildendes Oligonucleotid ist.

16. Verfahren zur Herstellung eines Invasoms gemäß einem der Ansprüche 1 bis 15, **dadurch gekennzeichnet, dass** das Lipidgemisch mit mindestens einem pharmakologischen Wirkstoff gemischt wird.

17. Arzneimittel enthaltend ein Invasom gemäß einem der Ansprüche 1 bis 15 und geeignete Hilfs- und Zusatzstoffe.

18. Verwendung eines Invasoms gemäß einem der Ansprüche 1 bis 15 zur Herstellung eines Medikaments zur Therapie einer Hauterkrankung.

19. Verwendung nach Anspruch 18, wobei die Hauterkrankung durch eine Modulation des Immunsystems behandelbar ist.

20. Verwendung nach Anspruch 19, wobei die Hauterkrankung Alopecia areata (alle klinischen Formen), Psoriasis vulgaris (alle klinischen Formen), atopische Dermatitis, atopisches Ekzem, Neurodermitis, polymorphe Lichtdermatose, Erythema solaris allergisches und irritatives Kontaktekzem, Arzneimittelexanthem, oder "Graft versus Host-Disease" ist.

21. Verwendung eines Invasoms gemäß einem der Ansprüche 1 bis 15 zur Herstellung eines Medikaments zur Therapie einer Erkrankung, die durch eine Modulation des Immunsystems behandelbar ist, insbesondere für eine prophylaktische und/oder therapeutische Vakzinierung

22. Verwendung nach Anspruch 21 wobei die Erkrankung eine Tumorerkrankung, Hyperplasie, proliferative Erkrankung, Arthritis, Viruserkrankung, bakteriellen und/oder parasitären Infektion ist.

23. Verwendung eines Invasoms enthaltend ein Lipidgemisch umfassend ein oder mehrere Lipide und ein oder mehrere Lysophosphatide, wobei die Lysophosphatide an dem Lipidgemisch einen Anteil im Bereich von 0,1 bis 40 Gew.-% haben, zur Herstellung eines Medikaments, das als Adjuvanz und/oder Trägersystem für Antigene und/oder andere immunmodulierende Moleküle bei der Behandlung einer Erkrankung, die durch eine Modulation des Immunsystems behandelbar ist, verwendbar ist.
